(19) Europäisches Patentamt
European Patent Office
Office européen des brevets

(11) EP 1 454 619 B1

(12) EUROPÄISCHE PATENTSCHRIFT

(45) Veröffentlichungstag und Bekanntmachung des Hinweises auf die Patenterteilung:
**20.02.2008 Patentblatt 2008/08**

(51) Int Cl.:
***A61Q 17/04*** (2006.01) ***A61Q 19/08*** (2006.01)
***A61K 8/06*** (2006.01) ***A61K 8/55*** (2006.01)

(21) Anmeldenummer: **04003572.7**

(22) Anmeldetag: **18.02.2004**

# (54) Kosmetische und dermatologische Emulsionen

Cosmetic and dermatological emulsions

Compositions cosmétiques et dermatologiques

(84) Benannte Vertragsstaaten:
**AT BE BG CH CY CZ DE DK EE ES FI FR GB GR HU IE IT LI LU MC NL PT RO SE SI SK TR**

(30) Priorität: **21.02.2003 DE 10307468**

(43) Veröffentlichungstag der Anmeldung:
**08.09.2004 Patentblatt 2004/37**

(73) Patentinhaber: **Beiersdorf AG**
**20245 Hamburg (DE)**

(72) Erfinder:
- **Eitrich, Anja**
**22587 Hamburg (DE)**
- **Grundt, Wiebke**
**20259 Hamburg (DE)**
- **von Bülow, Rixa, Dr.**
**20255 Hamburg (DE)**

(56) Entgegenhaltungen:
**EP-A- 0 754 446**
**EP-A- 1 310 239**
**US-A- 5 352 438**
**US-B1- 6 174 520**
**US-B1- 6 239 174**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft kosmetische und dermatologische Emulsionen mit einem Gehalt an organischen Feststoffen und Tensiden sowie ein Verfahren zur Stabilisierung von derartigen Emulsionen.

Kosmetische Zubereitungen werden im wesentlichen zur Pflege der Haut benutzt. Die menschliche Haut übt als größtes Organ des Menschen zahlreiche lebenswichtige Funktionen aus. Mit durchschnittlich etwa 2 $m^2$ Oberfläche beim Erwachsenen kommt ihr eine herausragende Rolle als Schutz- und Sinnesorgan zu. Unter ihren vielen Funktionen (beispielsweise zur Wärmeregulation) ist die Barrierefunktion, die das Austrocknen der Haut (und damit letztlich des gesamten Organismus) verhindert, die wohl wichtigste. Gleichzeitig wirkt die Haut als Schutzeinrichtung gegen das Eindringen und die Aufnahme von außen kommender Stoffe (z. B. Schmutz, Chemikalien, Mikroorganismen). Außerdem kommt ihr eine bedeutende Rolle als Regulations- und Zielorgan im menschlichen Stoffwechsel zu.

Die kosmetische Hautpflege dient in erster Linie dazu, die natürliche Funktion der Haut als Barriere gegen Umwelteinflüsse und gegen den Verlust von körpereigenen Stoffen (neben Wasser auch natürliche Fette, Elektrolyte etc.) zu stärken oder wiederherzustellen.

Ziel der Hautpflege ist es ferner, den durch tägliches Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Die schädigende Wirkung des ultravioletten Teils der Sonnenstrahlung auf die Haut ist allgemein bekannt. In Abhängigkeit von ihrer jeweiligen Wellenlänge haben die Strahlen verschiedene Wirkungen auf das Organ Haut: Die sogenannte UV-C-Strahlung mit einer Wellenlänge, die kleiner als 290 nm ist, wird von der Ozonschicht in der Erdatmosphäre absorbiert und hat keine physiologische Bedeutung. Dagegen verursachen Strahlen im Bereich zwischen 290 nm und 320 nm, dem sogenannten UV-B-Bereich, ein Erythem, einen einfachen Sonnenbrand oder sogar mehr oder weniger starke Verbrennungen. UV-A-Strahlung (320 bis 400 nm) ist im Hinblick auf die Auslösung photodynamischer, speziell phototoxischer Reaktionen und chronischer Veränderungen der Haut noch weitaus gefährlicher als UV-B-Strahlung. So reicht selbst die UV-A-Strahlung unter ganz normalen Alltagsbedingungen aus, um innerhalb kurzer Zeit die Kollagen- und Elastinfasern zu schädigen. Auch kann der schädigende Einfluss der UV-B-Strahlung durch UV-A-Strahlung noch verstärkt werden.

Ferner können bereits sehr geringe Strahlendosen photochemische Reaktionen auslösen. Hierzu gehört insbesondere die Bildung freier Radikale, welche wiederum aufgrund ihrer hohen Reaktivität unkontrollierte Folgereaktionen auslösen können. Um solchen Reaktionen vorzubeugen, können kosmetischen bzw. dermatologischen Formulierungen neben UV-Filtersubstanzen zusätzlich Antioxidantien und/oder Radikalfänger zugesetzt werden.

Medizinische topische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z. B. Kosmetikverordnung, Lebensmittel- und Arzneimittelgesetz).

Den bei weitem wichtigsten Produkttyp im Bereich der Hautpflegemittel stellen Emulsionen dar. Emulsionen sind disperse Zwei- oder Mehrphasensysteme, wobei kosmetische Emulsionen aus mindestens einer Fettphase (Fette und mineralische Öle, Fettsäureester, Fettalkohole etc.) und mindestens einer Wasserphase (Wasser, Glycerin, Glykole usw.) bestehen, die mit Hilfe von Emulgatoren in Form feinster Tröpfchen ineinander verteilt werden. Liegt die Ölphase fein verteilt in der Wasserphase vor, so handelt es sich um eine Öl-in-Wasser-Emulsion (O/W-Emulsion, z. B. Milch). Der Grundcharakter einer O/W-Emulsion ist durch das Wasser geprägt, d. h. sie wirkt weniger fettend auf der Haut, ist eher mattierend und zieht schneller in die Haut ein als eine W/O-Emulsion.

Obwohl Emulsionen vom thermodynamischen Standpunkt aus betrachtet instabile Systeme sind, gelingt es, kosmetische Emulsionen von jahrelanger Stabilität herzustellen. Eine Emulsion wird als stabil bezeichnet, wenn sich über einen vorgegebenen Zeitraum hinweg keine messbaren zeitlichen und örtlichen Änderungen der Tropfengrößenverteilung feststellen lassen.

Die Stabilität bzw. Instabilität von Emulsionen hängt von verschiedenen Faktoren ab. Zum einen neigt beispielsweise die Wasserphase einer W/O-Emulsion, da Wasser- und Ölphase unterschiedliche Dichten haben, zur Sedimentation. Die Ölphase einer O/W-Emulsion hat dementsprechend eine Tendenz zum Aufrahmen.

Ferner kann es aufgrund der Anziehungskräfte zwischen den feinverteilten Tröpfchen der dispersen Phase zu einer Tropfenaggregation kommen, wobei die einzelnen Tröpfchen eines Aggregates zunächst durch einen dünnen Film kontinuierlicher Phase voneinander getrennt bleiben. Die ursprüngliche Tropfengrößenverteilung ändert sich dabei nur scheinbar und kann in diesem Fall durch Rühren oder Schütteln wiederhergestellt werden.

Allerdings können die sich berührenden Tröpfchen darüber hinaus auch zusammenfließen, was zu einer echten Änderung der Tropfengrößenverteilung führt, die nur durch Energiezufuhr wieder verändert werden kann. Dieser Vorgang wird als Koaleszenz bezeichnet. Der Prozess der Koaleszenz läuft umso langsamer ab, je viskoser die äußere Phase der Emulsion ist.

Die beschriebenen Vorgänge können einzeln oder zusammen ablaufen. Oft initiiert oder verstärkt ein Vorgang den anderen. So wird z. B. durch die Bildung von Aggregaten in O/W-Emulsionen das Aufrahmen der Ölphase beschleunigt. Geht der disperse Zustand einer Emulsion teilweise oder auch ganz verloren, so trennen sich die beiden Phasen, und man spricht vom Brechen der Emulsion.

Zur Stabilisierung von Emulsionen über einen längeren Zeitraum hinweg werden dementsprechend Hilfsmittel benötigt, die das Entmischen der beiden Phasen unterbinden, mindestens aber so lange verzögern, bis die Emulsion ihre Bestimmung erfüllt hat.

Diese Hilfsmittel sollen zum einen die Grenzfläche stabilisieren, indem sie verhindern, daß die Tröpfchen der dispersen Phase zusammenfließen. Im Idealfall bewirken diese Stoffe darüber hinaus eine Abstoßung der Tröpfchen, welche verhindert, daß sich diese annähern, so daß eine Zusammenballung (Aggregatbildung) vermieden werden kann.

Zum anderen werden Hilfsstoffe dazu eingesetzt, dem Aufrahmen bzw. Sedimentieren der Phasen entgegenzuwirken.

Emulgatoren sind grenzflächenaktive Substanzen, die in der Lage sind, die Grenzflächenspannung zwischen Öl- und Wasserphase zu vermindern, indem sie sich bevorzugt an der Grenzfläche zwischen diesen beiden anlagern. Dies wird durch ihren amphipilen Molekülaufbau ermöglicht: Emulgatoren besitzen wenigstens eine polare (hydrophile) Gruppe und wenigstens eine unpolare (lipophile) Gruppe. Damit sind sie sowohl in der hydrophilen als auch in der lipophilen Phase löslich. Der in der entsprechenden Phase besser lösliche Teil ragt in diese hinein und senkt dadurch die Grenzflächenspannung zwischen beiden Phasen.

Der Versuch der Klassifizierung von Emulgatoren ist schwierig, da diese zu chemisch sehr unterschiedlichen Kategorien gehören. Ein Emulgator ist um so effektiver, je schneller er die Grenzflächenspannung erniedrigt und je niedriger der Gleichgewichtswert der Grenzflächenspannung ist.

Darüber hinaus stabilisieren Emulgatoren auch durch Ausbildung von Grenzflächenfilmen und damit "physikalischen" Barrieren, wodurch die Aggregatbildung und die Koaleszenz der emulgierten Teilchen verhindert wird. Durch die Anlagerung des Emulgators an der Grenzfläche werden die Tröpfchen entweder aufgeladen, so daß sie sich gegenseitig abstoßen, oder es wird eine stabile, vielfach hochviskose oder sogar feste Schutzschicht um die Tröpfchen gebildet.

Für die praktische Herstellung kosmetischer oder dermatologischer Emulsionen reicht allerdings in der Regel der Einsatz eines oder mehrerer Emulgatoren allein nicht aus. Wesentliche Faktoren für die Stabilität kosmetischer oder dermatologischer Zubereitungen sind ferner:

- feinste Verteilung der beiden Phasen ineinander Je kleiner die dispergierten Teilchen sind, umso stabiler ist die Emulsion.
- hohe Viskosität der äußeren Phase
- ein stabiler Grenzflächenfilm
- ein ausgewogenes Phasenvolumenverhältnis

Das Emulgatorsystem muss daher meist zusätzlich zum eigentlichen Emulgator noch eine weitere Komponente enthalten, die als Co-Emulgator, Stabilisator oder je nach Wirkmechanismus auch als Konsistenzgeber, Verdickungsmittel oder Schutzkolloid usw. bezeichnet wird.

Durch diese Stoffe, im folgenden der Einfachheit halber Stabilisatoren genannt, wird die Stabilität einer Emulsion erhöht. Stabilisatoren müssen nicht grenzflächenaktiv, können aber amphiphil aufgebaute Verbindungen sein.

Eine Möglichkeit, Emulsionen zu stabilisieren, ist nach dem oben gesagten, die Viskosität der äußeren Phase zu erhöhen. Diese Viskositätserhöhung bewirkt in der Regel eine erhebliche Verminderung der Beweglichkeit der dispergierten Tröpfchen, wodurch die Sedimentations- bzw. Aufrahmgeschwindigkeit vermindert wird. Damit einhergehend treffen die Tröpfchen ferner weniger häufig aufeinander, was eine geringere Koaleszenzneigung zur Folge hat.

Die Viskosität der äußeren Phase lässt sich beispielsweise durch Zugabe von Verdikkungsmitteln erhöhen, welche z. B. Gele und/oder lamellare Flüssigkristalle ausbilden. Auch Emulgatoren sind im Prinzip in der Lage, durch die Bildung von Emulgatorgelnetzwerken die Viskosität einer Flüssigkeit zu erhöhen. Allerdings wird hierfür eine vergleichsweise hohe Menge an Emulgator benötigt, da Gelnetzwerke erst dann gebildet werden, wenn die gesamte Grenzfläche zwischen den Phasen mit Emulgatormolekülen belegt ist.

Das Brechen einer Emulsion lässt sich ferner durch Wahl eines geeigneten Phasenvolumenverhältnisses verhindern. Zur Verdeutlichung dieser Tatsache stelle man sich eine Emulsion als System aus Metallkugeln gleichen Durchmessers (innere Phase) und einer Flüssigkeit (äußere Phase) vor. Eine Sedimentation bzw. ein Aufrahmen kann - in diesem einfachen Modell - dann nicht mehr stattfinden, wenn die gesamte Flüssigkeit mit Metallkugeln ausgefüllt ist. Dies ist - nimmt man eine dichteste Kugelpackung als Verteilung an - gerade bei einem Verhältnis von 1 : 2 der Fall, d. h. wenn die Emulsion zu 2/3 aus innerer Phase besteht. Es ist offensichtlich, daß die Viskosität einer Emulsion bei wachsendem Anteil an innerer Phase zunimmt, da hierdurch die Beweglichkeit der dispergierten Tröpfchen eingeschränkt wird.

Natürlich ist dem Fachmann eine Vielzahl von Möglichkeiten bekannt, stabile Emulsionen zur kosmetischen oder dermatologischen Anwendung zu formulieren, beispielsweise in Form von Cremes und Salben, die im Bereich von Raum- bis Hauttemperatur streichfähig sind, oder als Lotionen und Milche, die in diesem Temperaturbereich eher fließfähig sind. Dabei kommt es neben der Wahl des "richtigen" Emulgators bzw. Emulgatorsystems insbesondere auch auf die weitere Zusammensetzung der Zubereitung an.

Ein Nachteil von Emulsionen ist allerdings deren mangelnde Stabilität gegenüber höheren Konzentrationen an Tensiden, was sich beispielsweise in einer Phasentrennung äußert. Der Stand der Technik kennt zwar tensidhaltige Zubereitungen auf Emulsionsbasis. Allerdings werden diese üblicherweise in der Art formuliert, daß die Emulsion zunächst mit Emulgatoren stabilisiert und anschließend ein Tensidsystem angepasst wird. Da Emulsionen durch die Zugabe von Tensiden im allgemeinen zerstört werden, ist die Wahl des Tensidsystems stark eingeschränkt, und den erhaltenen Zubereitungen liegen teure und komplizierte Rezepturen zugrunde.

Oft wird die emulsionszerstörende Wirkung der Tenside durch weitere Bestandteile der Emulsion - wie beispielsweise (organische) Feststoffe und dergleichen - noch erhöht.

Es ist aber andererseits häufig wünschenswert, bestimmte Tenside einzusetzen, um deren sonstige physikalische, chemische bzw. physiologische Eigenschaften nutzen zu können.

Die Patentanmeldung US 5 352 438 offenbart kosmetische oder pharmazeutische O/W-Emulsionen enthaltend Dequest 2046, Cetylstearylalkohol, Stearylalkohol sowie UV-Filter. US 6 174 520 offenbart Sonnenschutzmittel in Form von O/W-Emulsion enthaltend Dequest 2046, UV-Filter in wäßriger Lösung und Tensid. EP 0 754 446 beschreibt Hautcreme in Form einer O/W-Emulsion enthaltend Glycerylstearat, Cetylalkohol, Dequest 2046 und einen partikelförmigen UV-Filter auf Titan- und Aluminiumsilkatbasis.

Eine Aufgabe der vorliegenden Erfindung war es also, kosmetische oder dermatologische Emulsionen - insbesondere O/W-Emulsionen - zu finden, welche gegenüber Tensiden - insbesondere in Kombination mit weiteren emulsionszerstörenden Stoffen - stabil sind.

Es hat sich überraschend gezeigt, und darin liegt die Lösung dieser Aufgaben, daß kosmetische und dermatologische Emulsionen, enthaltend

A. ein oder mehrere Tenside,
B. eine oder mehrere pigmentäre organische Substanzen und
C. Pentanatrium-Ethylendiamintetramethylenphosphonat den Nachteilen des Standes der Technik abhelfen würden.

Die Zubereitungen gemäß der vorliegenden Erfindung stellen in jeglicher Hinsicht überaus befriedigende Präparate dar, welche nicht auf eine eingeschränkte Rohstoffauswahl begrenzt sind. Dementsprechend eignen sie sich ganz besonders, um als Grundlage für Zubereitungsformen mit vielfältigen Anwendungszwecken zu dienen. Die Zubereitungen gemäß der vorliegenden Erfindung zeigen sehr gute sensorische und kosmetische Eigenschaften, wie beispielsweise die Verteilbarkeit auf der Haut oder das Einzugsvermögen in die Haut.

Erfindungsgemäß ist weiterhin auch die Verwendung von Pentanatrium-Ethyiendiamin-tetramethylenphosphonat zum Erzielen oder Erhöhen der Stabilität von Emulsionen mit einem Gehalt an pigmentären organischen Substanzen gegenüber der Gegenwart von Tensiden, insbesondere zum Erzielen oder Erhöhen der Stabilität von O/W-Emulsionen gegenüber der Gegenwart von Tensiden.

Erfindunsgemäß in weiterhin die Verwendung von Zubereitungen zur Herstellung einesKosmetikums zum Schutz der Haut vor vorzeitiger Alterung oder zur Hautbefeuchtung.

Pentanatrium-Ethylendiamintetramethylenphosphonat (INCI: Pentasodium Ethylenediamine Tetramethylene Phosphonate) hat die folgende Struktur

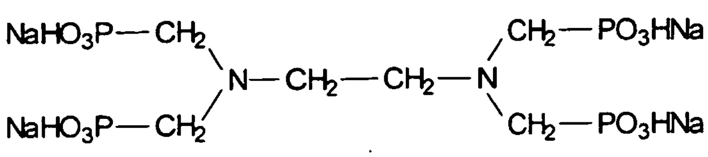

Es ist z. B. unter dem Handelsnamen Dequest 2046 von der Fa. Monsanto erhältlich.

Erfindungsgemäß enthalten kosmetische oder dermatologische Zubereitungen 0,001 bis 15 Gew.-%, vorteilhaft 0,005 bis 5 Gew.-%, ganz besonders bevorzugt 0,01 bis 1 Gew.-% Pentanatrium-Ethylendiamintetramethylenphosphonat.

Pigmentäre organische Substanzen (= organische Pigmente) im Sinne der vorliegenden Erfindung sind beispielsweise organische UV-Filtersubstanzen, die in der fertigen kosmetischen oder dermatologischen Zubereitung als

Feststoffe vorliegen.

Vorteilhaftes organisches Pigment im Sinne der vorliegenden Erfindung ist das 2,2'-Methylen-bis-(6-(2H-benzotriazol-2-yl)-4-(1,1,3,3-tetramethylbutyl)-phenol) [INCI: Methylene Bis-Benztriazolyl Tetramethylbutylphenol], welches durch die chemische Strukturformel

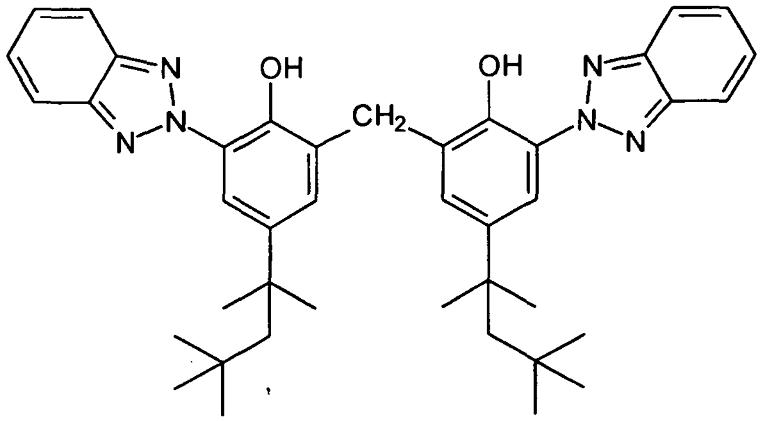

gekennzeichnet ist und unter der Handelsbezeichnung Tinosorb® M bei der CIBA-Chemikalien GmbH erhältlich ist.

Erfindungsgemäß enthalten kosmetische oder dermatologische Zubereitungen 0,001 bis 30 Gew.-%, vorteilhaft 0,05 bis 15 Gew.-%, ganz besonders bevorzugt 0,1 bis 10 Gew.-% pigmentären organischen Substanzen.

Mit "Tensid" werden im Sinne der vorliegenden Erfindung amphiphile Substanzen bezeichnet, welche organische, unpolare Substanzen in Wasser lösen können. Sie sorgen, bedingt durch ihren spezifischen Molekülaufbau mit mindestens einem hydrophilen und einem hydrophoben Molekülteil, für eine Herabsetzung der Oberflächenspannung des Wassers. Es gibt anionische, kationische, amphotere und nicht-ionische Tenside, die unterschiedliche funktionelle Gruppen aufweisen.

Anionische Tenside weisen als funktionelle Gruppen in der Regel Carboxylat-, Sulfat- oder Sulfonatgruppen auf. In wäßriger Lösung bilden sie im sauren oder neutralen Milieu negativ geladene organische Ionen. Kationische Tenside sind beinahe ausschließlich durch das Vorhandensein einer quarternären Ammoniumgruppe gekennzeichnet. In wäßriger Lösung bilden sie im sauren oder neutralen Milieu positiv geladene organische Ionen. Amphotere Tenside enthalten sowohl anionische als auch kationische Gruppen und verhalten sich demnach in wäßriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:

$$RNH_2^+CH_2CH_2COOH\ X^- \quad \text{(bei pH=2)}$$

$X^-$ = beliebiges Anion, z.B. $Cl^-$

$$RNH_2^+CH_2CH_2COO^- \quad \text{(bei pH=7)}$$

$$RNHCH_2CH_2COO^-\ B^+ \quad \text{(bei pH=12)}$$

$B^+$ = beliebiges Kation, z.B. $Na^+$

Typisch für nicht-ionische Tenside sind Polyether-Ketten. Nicht-ionische Tenside bilden in wäßrigem Medium keine Ionen.

Vorteilhaft im Sinne der vorliegenden Erfindung sind insbesondere nicht-ionische Tenside. Erfindgungsgemäß besonders vorteilhafte nicht-ionische Tenside sind durch folgende chemische Struktur charakterisiert

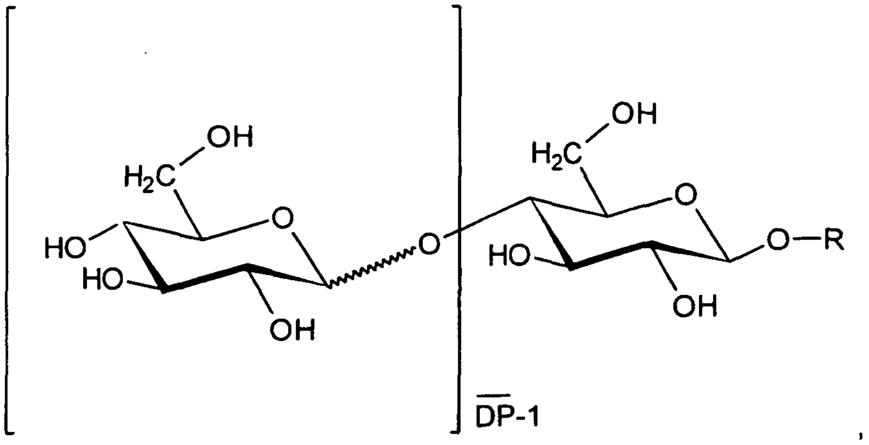

wobei R einen verzweigten oder unverzweigten Alkylrest mit 4 bis 24 Kohlenstoffatomen darstellt und wobei $\overline{DP}$ einen mittleren Glucosylierungsgrad von bis zu 2 bedeutet.

Der Wert $\overline{DP}$ repräsentiert den Glucosylierungsgrad der erfindungsgemäß verwendeten Alkylglucosiden und ist definiert als

$$\overline{DP} = \frac{p_1}{100} \cdot 1 + \frac{p_2}{100} \cdot 2 + \frac{p_3}{100} \cdot 3 + \ldots = \sum_i \frac{p_i}{100} \cdot i$$

Dabei stellen $p_1$, $p_2$, $p_3$ ... bzw, $p_i$ den Anteil der einfach, zweifach dreifach ... i-fach glucosylierten Produkte in Gewichtsprozenten dar. Erfindungsgemäß vorteilhaft werden Produkte mit Glucosylierungsgraden von 1 - 2, insbesondere vorteilhaft von 1,1 bis 1,5, ganz besonders vorteilhaft von ungefähr 1,3 gewählt.

Der Wert $\overline{DP}$ trägt dem Umstand Rechnung, daß Alkylglucoside herstellungsbedingt in der Regel Gemische aus Mono- und Oligoglucosiden darstellen. Erfindungsgemäß vorteilhaft ist ein relativ hoher Gehalt an Monoglucosiden, typischerweise in der Größenordnung von 40 bis 70 Gew.-%, bezogen auf das Gesamtgewicht des Gemisches.

Vorteilhaft wird R gewählt aus der Gruppe der unverzweigten Alkylreste, wobei der Myristylrest, der Cetylrest, der Stearylrest und der Eicosylrest bevorzugt werden. Bevorzugt sind Laurylglucosid und Cocoglucosid. Besonders bevorzugt ist Decylglucosid.

Erfindungsgemäß eingesetzte Alkylglucoside (auch: Alkylpolyglycoside) sind erhältlich durch Verfahren, wie sie beispielsweise in der DE-OS 40 40 655 und anderen Schriften beschrieben werden. Sie sind im Handel von verschiedenen Herstellern erhältlich.

Erfindungsgemäß enthalten kosmetische oder dermatologische Zubereitungen 0,001 bis 15 Gew.-%, vorteilhaft 0,005 bis 5 Gew.-%, ganz besonders bevorzugt 0,01 bis 1 Gew.-% an Tensiden.

Die Zubereitungen im Sinne der vorliegenden Erfindung können bevorzugt neben einer oder mehrerer Ölphasen zusätzlich eine oder mehrere Wasserphasen enthalten und beispielsweise in Form von W/O-, O/W-, W/O/W- oder O/W/O-Emulsionen vorliegen. Solche Formulierungen können vorzugsweise auch Mikroemulsionen, Stifte, Schäume (sog. Mousse), Feststoff-Emulsionen (d. h. Emulsionen, welche durch Feststoffe stabilisiert sind, z. B. Pickering-Emulsionen) oder sprühbare Emulsionen.

Auch (makroskopisch) zwei- oder mehrphasige Systeme sind erfindungsgemäß vorteilhaft. "Zwei- oder mehrphasig" im Sinne der vorliegenden Erfindung bedeutet, daß zwei oder mehr Phasen separat übereinander geschichtet vorliegen. Es ist besonders vorteilhaft im Sinne der vorliegenden Erfindung, wenn mindestens eine der makroskopisch sichtbaren Phasen eine (W/O-, O/W-, Mikro-) Emulsion darstellt. Die Emulsion wird bei dieser (makroskopischen) Betrachtung als eine Phase wahrgenommen, obwohl es dem Fachmann natürlich bekannt ist, daß Emulsionen an sich aus zwei oder mehr miteinander homogenisierten Phasen gebildet werden. Die "Emulsionsphase" ist langzeitstabil, so daß es auch über einen längeren Zeitraum (Monate, Jahre) nicht zu einer Entmischung beziehungsweise Phasenauftrennung innerhalb der Emulsion kommt.

Die makroskopisch sichtbaren Phasen bzw. Schichten lassen sich vorteilhaft - zum Beispiel durch Schütteln - kurzfristig zu einer homogenen Emulsion emulgieren, welche aber nicht langzeitstabil ist, sondern sich vielmehr über einen Zeitraum

von Minuten, Stunden oder Tagen wieder zu zwei oder mehr übereinander geschichteten Phasen entmischt. Es ist besonders vorteilhaft im Sinne der vorliegenden Erfindung, wenn mindestens eine der makroskopisch sichtbaren Phasen eine Mikroemulsion und mindestens eine andere der makroskopisch sichtbaren Phasen eine Ölphase darstellt.

**Sprühbare Emulsionen, insbesondere Mikroemulsionen**

Besonders vorteilhaft im Sinne der vorliegenden Erfindung sind sprühbare O/W-Emulsionen, insbesondere O/W-Mikroemulsionen.

Die Tröpfchendurchmesser der gewöhnlichen "einfachen", also nichtmultiplen Emulsionen liegen im Bereich von ca 1 μm bis ca. 50 μm. Solche "Makroemulsionen" sind, ohne weitere färbende Zusätze, milchigweißgefärbt und opak. Feinere "Makroemulsionen", deren Tröpfchendurchmesser im Bereich von ca. 0,5 μm bis ca. 1 μm liegen, sind, wiederum ohne färbende Zusätze, bläulichweißgefärbt und opak. Solche "Makroemulsionen" haben für gewöhnlich ein hohe Viskosität.

Der Tröpfchendurchmesser von Mikroemulsionen im Sinne der vorliegenden Erfindung dagegen liegt im Bereich von etwa 50 bis etwa 500 nm. Derartige Mikroemulsionen sind bläulichweiß gefärbt bis transluzent und meist niedrigviskos. Die Viskosität vieler Mikroemulsionen vom O/W-Typ ist vergleichbar mit der des Wassers.

Vorteil von Mikroemulsionen ist, daß in der dispersen Phase Wirkstoffe wesentlich feiner dispers vorliegen können als in der dispersen Phase von "Makroemulsionen". Ein weiterer Vorteil ist, daß sie aufgrund ihrer niedrigen Viskosität versprühbar sind. Werden Mikroemulsionen als Kosmetika verwendet, zeichnen sich entsprechende Produkte durch hohe kosmetische Eleganz aus.

Erfindungsgemäß vorteilhaft sind insbesondere O/W-Mikroemulsionen, welche mit Hilfe der sogenannten Phaseninversionstemperatur-Technologie erhältlich sind und mindestens einen Emulgator (Emulgator A) enthalten, welcher gewählt wird aus der Gruppe der Emulgatoren mit folgenden Eigenschaften:

- ihre Lipophilie ist abhängig von der Temperatur, dergestalt daß durch Erhöhung der Temperatur die Lipophilie zunimmt und durch Senkung der Temperatur die Lipophilie des Emulgators abnimmt.

Vorteilhafte Emulgatoren A sind z. B. polyethoxilierte Fettsäuren (PEG-100 Stearat, PEG-20 Stearat, PEG-150 Laurath, PEG-8 Distearat und dergleichen) und/oder polyethoxilierte Fettalkohole (Ceteareth-12, Ceteareth-20, Isoceteth-20, Beheneth-20, Laureth-9 etc.) und/oder Alkylpolyglycoside (Cetearyl Glycoside, Stearyl Glycoside, Palmityl Glycoside etc.).

Sofern die Phaseninversion im wesentlichen durch Variation der Temperatur eingeleitet wird, sind O/W-Emulsionen, insbesondere O/W-Mikroemulsionen erhältlich, wobei die Größe der Öltröpfchen im wesentlichen durch die Konzentration des oder der eingesetzten Emulgatoren bestimmt wird, dergestalt, daß eine höhere Emulgatorkonzentration kleinere Tröpfchen bewirkt und geringere Emulgatorkonzentration zu größeren Tröpfchen führt. Die Tröpfchengrößen liegen in der Regel zwischen 20 und 500 nm.

Es ist im Sinn der vorliegenden Erfindung gegebenenfalls vorteilhaft, weitere, nicht unter die Definition des Emulgators A fallende, W/O und/oder O/W-Emulgatoren zu verwenden, beispielsweise um die Wasserfestigkeit der Zubereitungen gemäß der vorliegenden Erfindung zu erhöhen. Hier können z. B. Alkylmethiconcopolyole und/oder Alkyl-Dimethiconcopolyole (insbesondere Cetyl Dimethicone Copolyol, Lauryl Methicone Copolyol), W/O-Emulgatoren (wie z. B. Sorbitanstearat, Glycerylstearat, Glycerolstearat, Sorbitanoleat, Lecithin, Glycerylisostearat, Polyglyceryl-3-Oleat, Polyglyceryl-3 Diisostearat, PEG-7-hydriertes Ricinusöl, Polyglyceryl-4-isostearat, Acrylat/ $C_{10-30}$-Alkylacrylat-Crosspolymer, Sorbitanisostearat, Poloxamer 101, Polyglyceryl-2-dipolyhydroxystearat, Polyglyceryl-3-Diisostearat, Polyglyceryl-4-dipolyhydroxystearat, PEG-30-dipolyhydroxystearat, Diisostearoylpolyglyceryl-3-diisostearat, Glycol Distearat, Polyglyceryl-3-dipolyhydroxystearat) und/oder Fettsäureester der Schwefel- oder Phosphorsäure (Cetylphosphat, Trilaureth-4 Phosphat, Trioleth-8-Phosphat, Stearylphosphat, Cetearylsulfat etc.) verwendet werden.

Weitere vorteilhafte sprühbare O/W-Emulsionen im Sinne der vorliegenden Erfindung sind dünnflüssige kosmetische oder dermatologische Hydrodispersionen, welche mindestens eine Ölphase und mindestens eine Wasserphase enthalten, wobei die Zubereitung durch mindestens einen Gelbildner stabilisiert wird und nicht notwendigerweise Emulgatoren enthalten muß, aber einen oder mehrere Emulgatoren enthalten kann.

Vorteilhafte Gelbildner für derartige Zubereitungen sind beispielsweise Copolymere aus $C_{10-30}$-Alkylacrylaten und einem oder mehreren Monomeren der Acrylsäure, der Methacrylsäure oder deren Ester. Die INCI-Bezeichnung für solche Verbindungen ist "Acrylates/C 10-30 Alkyl Acrylate Crosspolymer". Insbesondere vorteilhaft sind die Pemulen® Typen TR 1, TR 2 und TRZ von der Fa. Goodrich (Noveon).

Auch Carbopole sind vorteilhafte Gelbildner für derartige Zubereitungen. Carbopole sind Polymere der Acrylsäure, insbesondere auch Acrylat-Alkylacrylat-Copolymere. Vorteilhafte Carbopole sind beispielsweise die Typen 907, 910, 934, 940, 941, 951, 954, 980, 981, 1342, 1382, 2984 und 5984. ebenso die ETD-Typen 2020, 2050 und Carbopol Ultrez 10. Weitere vorteilhafte Gelbildner für derartige Zubereitungen sind Xanthan Gummi, Cellulose Derivate und

Johannisbrotkernmehl.

Als mögliche (fakultative) Emulgatoren können ethoxilierte Fettalkohole oder ethoxilierte Fettsäuren (insbesondere PEG-100 Stearat, Ceteareth-20) und/oder andere nichtionische oberflächenaktive Substanzen verwendet werden.

Ferner vorteilhaft können die sehr dünnflüssigen bis sprühbaren Emulsionen auch W/O- bzw. Wasser-in-Silikonöl- (W/S-) Emulsionen sein. Insbesondere vorteilhaft sind W/O- bzw. W/S-Emulsionen, die

- mindestens einen Silikonemulgator (W/S) mit einem HLB-Wert ≤ 8 und/oder mindestens einen W/O-Emulgator mit einem HLB-Wert < 7 und
- mindestens einen O/W-Emulgator mit einem HLB-Wert > 10 enthalten.

Derartige Zubereitungen enthalten ferner mindestens 20 Gew.-% Lipide, wobei die Lipidphase vorteilhaft auch Silikonöle enthalten bzw. sogar ganz aus solchen bestehen kann.

Der oder die Silikonemulgatoren kann vorteilhaft aus der Gruppe der Alkylmethiconcopolyole und/oder Alkyl-Dimethiconcopolyole gewählt werden (z. B. Dimethiconcopolyole, welche von der Goldschmidt AG unter den Warenbezeichnungen ABIL® B 8842, ABIL® B 8843, ABIL® B 8847, ABIL® B 8851, ABIL® B 8852, ABIL® B 8863, ABIL® B 8873 und ABIL® B 88183 verkauft werden, Cetyl Dimethiconcopolyol [Goldschmidt AG / ABIL® EM 90], Cyclomethicon Dimethiconcopolyol [Goldschmidt AG / ABIL® EM 97], Laurylmethiconcopolyol [Dow Corning Ltd. / Dow Corning® 5200 Formulation Aid], Octyl Dimethicon Ethoxy Glucosid [Firma Wacker].

Der oder die W/O-Emulgatoren mit einem HLB-Wert < 7 kann vorteilhaft aus folgender Gruppe gewählt werden: Sorbitanstearat, Sorbitanoleat, Lecithin, Glyceryllanolat, Lanolin, hydriertem Ricinusöl, Glycerylisostearat, Polyglyceryl-3-Oleat, Pentaerythrithylisostearat, Methylglucosedioleat, Methylglucosedioleat im Gemisch mit Hydroxystearat und Bienenwachs, PEG-7-hydriertes Ricinusöl, Polyglyceryl-4-isostearat, Hexyllaurat, Acrylat/ $C_{10-30}$-Alkylacrylat-Crosspolymer, Sorbitanisostearat, Polyglyceryl-2-dipolyhydroxystearat, Polyglyceryl-3-Diisostearat, PEG-30-dipolyhydroxystearat, Diisostearoylpolyglyceryl-3-diisostearat, Polyglyceryl-3-dipolyhydroxystearat, Polyglyceryl-4-dipolyhydroxystearat, Polyglyceryl-3-dioleat.

Der oder die O/W-Emulgatoren mit einem HLB-Wert > 10 kann vorteilhaft aus folgender Gruppe gewählt werden: Glycerylstearat im Gemisch mit Ceteareth-20, Ceteareth-25, Ceteareth-6 im Gemisch mit Stearylalkohol, Cetylstearylalkohol im Gemisch mit PEG-40-Ricinusöl und Natriumcetylstearylsulfat, Triceteareth-4 Phosphat, Glycerylstearat, Natriumcetylstearylsulfat, Lecithin Trilaureth-4 Phosphat, Laureth-4 Phosphat, Stearinsäure, Propylenglycolstearat SE, PEG-9-Stearat, PEG-20 Stearat, PEG-30-Stearat, PEG-40-Stearat, PEG-100-Stearat, Ceteth-2, Ceteth-20, Polysorbate-20, Polysorbate-60, Polysorbate-65, Polysorbate-100, Glycerylstearat im Gemisch mit PEG-100 Stearat, Ceteareth-3, Isostearylglycerylether, Cetylstearylalkohol im Gemisch mit Natrium Cetylstearylsulfat, PEG-40-Stearat, Glycol Distearat, PEG-22-Dodecyl Glycol Copolymer, Polyglyceryl-2-PEG-4-Stearat, Ceteareth-12, Ceteareth-20, Ceteareth-30, Methyl-glucosesesquistearat, Steareth-10, PEG-20-Stearat, Steareth-21, Steareth-20, Isosteareth-20, PEG-45/Dodecylglycol-Copolymer, Methoxy-PEG-22/Dodecylglycol-Copolymer, Glycerylstearat SE, Ceteth-20, PEG-20-Methylglucosesesquistearat, Glycerylstearatcitrat, Cetylphosphat, Cetearyl Sulfat, Sorbitansesquioleat, Triceteareth-4-Phosphat, Trilaureth-4-Phosphat, Polyglyceryl-methylglucosedistearat, Kaliumcetylphosphat, Isosteareth-10, Polyglyceryl-2-sesquiisostearat, Ceteth-10, Isoceteth-20, Glycerylstearat im Gemisch mit Ceteareth-20, Ceteareth-12, Cetylstearylalkohol und Cetylpalmitat, PEG-30-Stearat, PEG-40-Stearat, PEG-100-Stearat.

Die Zubereitungen gemäß der vorliegenden Erfindung können vorteilhaft auch als kosmetische oder dermatologische Tränkungslösungen, mit welchen insbesondere wasserunlösliche Substrate - wie z. B. gewebte oder nichtgewebte Tücher - befeuchtet sind, verwendet werden. Derartige Tränkungslösungen sind vorzugsweise dünnflüssig, insbesondere sprühbar (wie z. B. PIT-Emulsionen, W/O-Emulsionen etc.) und haben vorzugsweise eine Viskosität von weniger als 2000 mPa·s, insbesondere weniger als 1.500 mPa·s (Meßgerät: Haake Viskotester VT-02 bei 25 °C). Mit ihrer Hilfe sind beispielsweise kosmetische Sonnenschutztücher, Pflegetücher und dergleichen erhältlich, welche die Kombination eines weichen, wasserunlöslichen Materials mit der dünnflüssigen kosmetischen und dermatologischen Tränkungslösung darstellen.

## Schäume

Besonders vorteilhaft im Sinne der vorliegenden Erfindung sind ferner selbstschäumende, schaumförmige, nachschäumende oder schäumbare kosmetische und dermatologische Zubereitungen.

Unter "selbstschäumend", schaumförmig", "nachschäumend" bzw. "schäumbar" sind Zubereitungen zu verstehen, aus welchen Schäume - sei es bereits während des Herstellprozesses, sei es bei der Anwendung durch den Verbraucher oder auf andere Weise

- durch Eintrag eines oder mehrerer Gase im Prinzip herstellbar sind. In derartigen Schäumen liegen die Gasbläschen

(beliebig) verteilt in einer (oder mehreren) flüssigen Phase(n) vor, wobei die (aufgeschäumten) Zubereitungen makroskopisch nicht notwendigerweise das Aussehen eines Schaumes haben müssen. Erfindungsgemäße (aufgeschäumte) kosmetische oder dermatologische Zubereitungen (im folgenden der Einfachheit halber auch als Schäume bezeichnet) können z. B. makroskopisch sichtbar dispergierte Systeme aus in Flüssigkeiten dispergierten Gasen darstellen. Der Schaumcharakter kann aber beispielsweise auch erst unter einem (Licht-) Mikroskop sichtbar werden. Darüber hinaus sind erfindungsgemäße Schäume - insbesondere dann, wenn die Gasbläschen zu klein sind, um unter einem Lichtmikroskop erkannt zu werden - auch an der starken Volumenzunahme des Systems erkennbar.

Deratige Zubereitungen enthalten im Sinne der vorliegenden Erfindung vorteilhaft ein Emulgatorsystem, welches aus

A. mindestens einem Emulgator A, gewählt aus der Gruppe der ganz-, teil- oder nicht neutralisierten, verzweigten und/oder unverzweigten, gesättigten und/oder ungesättigten Fettsäuren mit einer Kettenlänge von 10 bis 40 Kohlenstoffatomen,
B. mindestens einem Emulgator B, gewählt aus der Gruppe der polyethoxylierten Fettsäurester mit einer Kettenlänge von 10 bis 40 Kohlenstoffatomen und mit einem Ethoxylierungsgrad von 5 bis 100 und
C. mindestens einem Coemulgator C, gewählt aus der Gruppe der gesättigten und/oder ungesättigten, verzweigten und/oder unverzweigten Fettalkohole mit einer Kettenlänge von 10 bis 40 Kohlenstoffatomen besteht.

Der oder die Emulgatoren A werden vorzugsweise gewählt aus der Gruppe der Fettsäuren, welche ganz oder teilweise mit üblichen Alkalien (wie z. B. Natrium- und/oder Kaliumhydroxid, Natrium- und/oder Kaliumcarbonat sowie Mono- und/oder Triethanolamin) neutralisiert sind. Besonders vorteilhaft sind beispielsweise Stearinsäure und Stearate, Isostearinsäure und Isostearate, Palmitinsäure und Palmitate sowie Myristinsäure und Myristate.

Der oder die Emulgatoren B werden vorzugsweise gewählt aus der folgenden Gruppe: PEG-9-Stearat, PEG-8-Distearat, PEG-20-Stearat, PEG-8 Stearat, PEG-8-Oleat, PEG-25-Glyceryltrioleat, PEG-40-Sorbitanlanolat, PEG-15-Glycerylricinoleat, PEG-20-Glycerylstearat, PEG-20-Glycerylisostearat, PEG-20-Glyceryloleat, PEG-20-Stearat, PEG-20-Methylglucosesesquistearat, PEG-30-Glycerylisostearat, PEG-20-Glyceryllaurat, PEG-30-Stearat, PEG-30-Glycerylstearat, PEG-40-Stearat, PEG-30-Glyceryllaurat, PEG-50-Stearat, PEG-100-Stearat, PEG-150-Laurat. Besonders vorteilhaft sind beispielsweise polyethoxylierte Stearinsäureester.

Der oder die Coemulgatoren C werden erfindungsgemäß vorzugsweise aus der folgenden Gruppe gewählt: Behenylalkohol ($C_{22}H_{45}OH$), Cetearylalkohol [eine Mischung aus Cetylalkohol ($C_{16}H_{33}OH$) und Stearylalkohol ($C_{18}H_{37}OH$)], Lanolinalkohole (Wollwachsalkohole, die die unverseifbare Alkoholfraktion des Wollwachses darstellen, die nach der Verseifung von Wollwachs erhalten wird). Besonders bevorzugt sind Cetyl- und Cetylstearylalkohol.

Es ist erfindungsgemäß vorteilhaft, die Gewichtsverhältnisse von Emulgator A zu Emulgator B zu Coemulgator C (A : B : C) wie a : b : c zu wählen, wobei a, b und c unabhängig voneinander rationale Zahlen von 1 bis 5, bevorzugt von 1 bis 3 darstellen können. Insbesondere bevorzugt ist ein Gewichtsverhältnis von etwa 1 : 1 : 1.

Es ist vorteilhaft im Sinne der vorliegenden Erfindung, die Gesamtmenge der Emulgatoren A und B und des Coemulgators C aus dem Bereich von 2 bis 20 Gew.-%, vorteilhaft von 5 bis 15 Gew.-%, insbesondere von 7 bis 13 Gew.-%, jeweils bezogen auf das Gesamtgewicht der Formulierung, zu wählen.

## Pickering-/ feststoffstabilisierte Emulsionen

Besonders vorteilhaft im Sinne der vorliegenden Erfindung sind ferner kosmetische oder dermatologische Zubereitungen, welche nur durch feinstverteilte Feststoffteilchen stabilisiert sind. Solche "emulgatorfreien" Emulsionen werden auch als Pickering-Emulsionen bezeichnet.

In Pickering-Emulsionen kommt es zu einer Anreicherung des festen Stoffes an der Phasengrenze Öl/Wasser in Form einer Schicht, wodurch ein Zusammenfließen der dispersen Phasen verhindert wird. Von wesentlicher Bedeutung sind dabei insbesondere die Oberflächeneigenschaften der Feststoffpartikel, welche sowohl hydrophile als auch lipophile Eigenschaften zeigen sollten.

Vorteilhaft können die stabilisierenden Feststoffteilchen auch oberflächlich wasserabweisend behandelt ("gecoatet") sein, wobei ein amphiphiler Charakter dieser Feststoffteilchen gebildet werden bzw. erhalten bleiben soll. Die Oberflächenbehandlung kann darin bestehen, daß die Feststoffteilchen nach an sich bekannten Verfahren mit einer dünnen hydrophoben bzw. hydrophilen Schicht versehen werden.

Der mittlere Partikeldurchmesser der als Stabilisator verwendeten mikrofeinen Feststoffteilchen wird vorzugsweise kleiner als 100 $\mu$m, besonders vorteilhaft kleiner als 50 $\mu$m gewählt. Dabei ist es im wesentlichen unerheblich, in welcher Form (Plättchen, Stäbchen, Kügelchen etc.) bzw. Modifikation die verwendeten Feststoffteilchen vorliegen.

Vorzugsweise werden die mikrofeinen Feststoffteilchen aus der Gruppe der amphiphilen Metalloxidpigmente gewählt. Vorteilhaft sind insbesondere:

- Titandioxide (gecoatet und ungecoatet): z. B. Eusolex T-2000 von der Fa. Merck, Titandioxid MT-100 Z von der Fa. Tayca Corporation
- Zinkoxide z. B. Z-Cote und Z-Cote HP1 von der BASF AG, MZ -300, MZ -500 und MZ-505M von der Fa. Tayca Corporation
- Eisenoxide

Des weiteren ist es vorteilhaft, wenn die mikrofeinen Feststoffteilchen aus der folgenden Gruppe gewählt werden: Bornitride, Stärkederivate (Tapioca Starch, Sodium Corn Starch Octynylsuccinat etc.), Talkum, Latexpartikel.

Es ist erfindungsgemäß vorteilhaft, wenn die feststoffstabilisierten Emulsionen deutlich weniger als 0,5 Gew.-% eines oder mehrerer Emulgatoren enthalten bzw. sogar gänzlich emulgatorfrei sind.

**Stifte**

Auch wasserhaltige stiftförmige Zubereitungen sind an sich bekannt, wobei diese auch in Form von W/O-Emulsionen vorliegen können.

**Anwendung und weitere Inhaltsstoffe:**

Die erfindungsgemäßen kosmetischen oder dermatologischen Formulierungen können wie üblich zusammengesetzt sein und der kosmetischen oder dermatologischen Lichtschutz, ferner zur Behandlung, Pflege und Reinigung der Haut und/oder der Haare und als Schminkprodukt in der dekorativen Kosmetik dienen.

Entsprechend ihrem Aufbau können kosmetische oder topische dermatologische Zusammensetzungen im Sinne der vorliegenden Erfindung, beispielsweise verwendet werden als Hautschutzcrème, Reinigungsmilch, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Zur Anwendung werden die kosmetischen und dermatologischen Zubereitungen in der für Kosmetika üblichen Weise auf die Haut und/oder die Haare in ausreichender Menge aufgebracht.

Die kosmetischen und dermatologischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Komplexbildner, Bakterizide, Parfüme, Substanzen zum Verhindern oder Steigern des Schäumens, Farbstoffe, Pigmente, die eine färbende Wirkung haben, Verdickungsmittel, anfeuchtende und/oder feuchhaltende Substanzen, Füllstoffe, die das Hautgefühl verbessern, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Vorteilhafte Konservierungsmittel im Sinne der vorliegenden Erfindung sind beispielsweise Formaldehydabspalter (wie z. B. DMDM Hydantoin, welches beispielsweise unter der Handelsbezeichnung Glydant™ von der Fa. Lonza erhältlich ist), Iodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Octoxyglycerin, Glycine Soja etc.

Vorteilhafte Komplexbildner im Sinne der vorliegenden Erfindung sind beispielsweise EDTA, [S,S]-Ethylendiamindisuccinat (EDDS), welches beispielsweise unter der Handelsbezeichnung Octaquest von der Fa. Octel erhältlich ist und/oder Iminodibersteinsäure, welche u. a. von der Fa. Bayer AG unter den Handelsnamen Iminodisuccinat VP OC 370 (ca. 30% ige Lösung) und Baypure CX 100 fest erhältlich ist.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist insbesondere vorteilhaft, wenn die kosmetischen Zubereitungen gemäß der vorliegenden Erfindung kosmetische oder dermatologische Wirkstoffe enthalten, wobei bevorzugte Wirkstoffe Antioxidantien sind, welche die Haut vor oxidativer Beanspruchung schützen können.

Weitere vorteilhafte Wirkstoffe im Sinne der vorliegenden Erfindung sind natürliche Wirkstoffe und/oder deren Derivate, wie z. B. alpha-Liponsäure, Phytoen, D-Biotin, Coenzym Q10, alpha-Glucosylrutin, Carnitin, Carnosin, natürliche und/oder synthetische Isoflavonoide, Kreatin, Taurin und/oder β-Alanin sowie 8-Hexadecen-1,16-dicarbonsäure (Dioic acid, CAS-Nummer 20701-68-2; vorläufige INCI-Bezeichnung Octadecendioic acid).

Erfindungsgemäße Rezepturen, welche z. B. bekannte Antifaltenwirkstoffe wie Flavonglycoside (insbesondere α-Glycosylrutin), Coenzym Q10, Vitamin C und/oder Derivate, Vitamin A und/oder Derivate, Vitamin E und/oder Derivate und dergleichen enthalten, eignen sich insbesondere vorteilhaft zur Prophylaxe und Behandlung kosmetischer oder dermatologischer Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Cuperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissigkeit) und dergleichen). Weiterhin vorteilhaft eignen sie sich gegen das Erscheinungsbild der trockenen bzw. rauhen Haut.

Die Wasserphase der Zubereitungen gemäß der vorliegenden Erfindung kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Butylenglykol, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole [von der Fa. Bf. Goodrich], beispielsweise Carbopole der Typen 980, 981, 1382, 2984, 5984, ETD 2020, ETD 2050, Ultrez 10, jeweils einzeln oder in Kombination.

Die Zubereitungen gemäß der vorliegenden Erfindung können ferner vorteilhaft auch Selbstbräunungssubstanzen enthalten, wie beispielsweise Dihydroxyacteon und/oder Melaninderivate in Konzentrationen von 1 Gew.-% bis zu 8 Gew.-%, bezogen auf das Gesamtgewicht der Zubereitung.

Ferner vorteilhaft können die Zubereitungen gemäß der vorliegenden Erfindung auch Repellentien zum Schutz vor Mücken, Zecken und Spinnen und dergleichen enthalten. Vorteilhaft sind z. B. N,N-Diethyl-3-methylbenzamid (Handelsbezeichnung: Metadelphene, "DEET"), Dimethylphtalat (Handelsbezeichnung: Palatinol M, DMP) sowie insbesondere 3-(N-n-Butyl-N-acetyl-amino)-propionsäureethylester (unter dem Handelsnamen Insekt Repellent® 3535 bei der Fa. Merck erhältlich). Die Repellentien können sowohl einzeln als auch in Kombination eingesetzt werden.

Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Moisturizer im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zur Prophylaxe und Behandlung kosmetischer oder dermatologischer Hautveränderungen, wie sie z. B. bei der Hautalterung auftreten, verwendet werden.

Die erfindungsgemäßen kosmetischen oder dermatologischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile® (CAS-Nr. 7631-86-9).

Die Ölphase der erfindungsgemäßen Formulierungen wird vorteilhaft gewählt aus der Gruppe der polaren Öle, beispielsweise aus der Gruppe der Lecithine und der Fettsäuretriglyceride, namentlich der Triglycerinester gesättigter

und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche.

Es ist ferner bevorzugt, Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, $C_{12-13}$-Alkyllactat, Di-$C_{12-13}$-Alkyltartrat, Triisostearin, Dipentaerythrityl Hexacaprylat/Hexacaprat, Propylenglykolmonoisostearat, Tricaprylin, Dimethylisosorbid einzusetzen. Es ist insbesondere vorteilhaft, wenn die Ölphase der erfindungsgemäßen Formulierungen einen Gehalt an $C_{12-15}$-Alkylbenzoat aufweist.

Vorteilhafte Ölkomponenten sind ferner z. B. Butyloctylsalicylat (beispielsweise das unter der Handelsbezeichnung *Hallbrite BHB* bei der Fa. CP Hall erhältliche), Hexadecylbenzoat und Butyloctylbenzoat und Gemische davon (*Hallstar AB*) und/oder Diethylhexylnaphthalat (*Hallbrite TQ oder Corapan TQ von H&R*).

Auch beliebige Abmischungen solcher Öl- und Wachskomponenten sind vorteilhaft im Sinne der vorliegenden Erfindung einzusetzen.

Besonders vorteilhafte Wachskomponenten, die die Wasserfestigkeit speziell von Lichtschutzformulierungen zusätzlich steigern können, sind ferner acetyliertes Glykolstearat mit Tristearin (z. B. Unitwix von ISP mit der INCI: Acetylated Glycol Stearat and Tristearin), $C_{18-36}$ Fettsäuretriglycerid (z. B: Syncrowax HGLC von der Fa. Crode GmbH mit der INCI: C18-36 Acid Triglyceride) sowie die unter den Handelsbezeichnungen "Performa V 1608" (INCI: C30-38 Olefin/Isopropyl Maleate/MA Copolymer) und "Perfroma V 825" (synethisches Wachs) von New Phase Technologies erhältlichen Substanzen.

Weitere vorteilhafte Substanzen, welche in die Ölphase der Zubereitungen gemäß der vorliegenden Erfindung eingearbeitet werden und zur Steigerung der Wasserfestigkeit beitragen können, sind PEG-45 Dodecylglykolcopolymer (INCI: PEG-45 Dodecyl Glycol Copolymer) und Methoxy PEG-22 Dodecylglykolcopolymer (INCI: Methoxy PEG-22 Dodecyl Glycol Copolymer), welche beide von der Firma AKZO Nobel erhältlich sind.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Silikonöle sind hochmolekulare synthetische polymere Verbindungen, in denen SiliciumAtome über Sauerstoff-Atome ketten- und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel auszeichnen

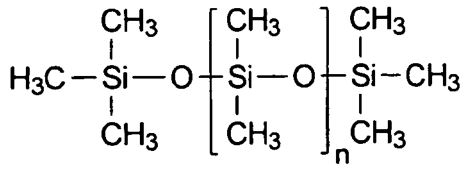

werden auch als Polydimethylsiloxan bzw. Dimethicon (INCI) bezeichnet. Dimethicone gibt es in verschiedenen Kettenlängen bzw. mit verschiedenen Molekulargewichten.

Besonders vorteilhafte Polyorganosiloxane im Sinne der vorliegenden Erfindung sind beispielsweise Dimethylpolysiloxane [Poly(dimethylsiloxan)], welche beispielsweise unter den Handelsbezeichnungen Abil 10 bis 10 000 bei Th. Goldschmidt erhältlich sind. Ferner vorteilhaft sind Phenylmethylpolysiloxane (INCI: Phenyl Dimethicone, Phenyl Trimethicone), cyclische Silikone (Octamethylcyclotetrasiloxan bzw. Decamethylcyclopentasiloxan), welche nach INCI auch als Cyclomethicone bezeichnet werden, aminomodifizierte Silikone (INCI: Amodimethicone) und Silikonwachse, z. B. Polysiloxan-Polyalkylen-Copolymere (INCI: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan).

Die Zubereitungen gemäß der vorliegenden Erfindung können ferner vorteilhaft eine oder mehrere Substanzen aus der folgenden Gruppe der Siloxanelastomere enthalten, beispielsweise um die Wasserfestigkeit und/oder den Lichtschutzfaktor der Produkte zu steigern:

(a) Siloxanelastomere, welche die Einheiten $R_2SiO$ und $RSiO_{1,5}$ und/oder $R_3SiO_{0.5}$ und/oder $SiO_2$ enthalten, wobei die einzelnen Reste R jeweils unabhängig voneinander Wasserstoff, $C_{1-24}$-Alkyl (wie beispielsweise Methyl, Ethyl, Propyl) oder Aryl (wie beispielsweise Phenyl oder Tolyl), Alkenyl (wie beispielsweise Vinyl) bedeuten und das Gewichtsverhältnis der Einheiten $R_2SiO$ zu $RSiO_{1,5}$ aus dem Bereich von 1 : 1 bis 30 : 1 gewählt wird;

(b) Siloxanelastomere, welche in Silikonöl unlöslich und quellfähig sind, die durch die Additionsreaktion eines Organopolysiloxans (1), das siliciumbebundenen Wasserstoff enthält, mit einem Organopolysiloxan (2), das ungesättigte aliphatische Gruppen enthält, erhältlich sind,

wobei die verwendeten Mengenateile so gewählt werden, daß die Menge des Wasserstoffes des Organopolysiloxans (1) oder der ungesättigten aliphatischen Gruppen des Organopolysiloxans (2)

- im Bereich von 1 bis 20 mol-% liegt, wenn das Organopolysiloxan nicht cyclisch ist und
- im Bereich von 1 bis 50 mol-% liegt, wenn das Organopolysiloxan cyclisch ist.

Vorteilhaft im Sinne der vorliegenden Erfindung liegen das oder die Siloxanelastomere in Form sphärischer Puder oder in Form von Gelen vor.

Erfindungsgemäß vorteilhafte in Form sphärischer Puder vorliegende Siloxanelastomere sind die mit der INCI-Bezeichnung Dimethicone / Vinyl Dimethicone Crosspolymer, beispielsweise das von DOW CORNING unter der Handelsbezeichnungen DOW CORNING 9506 Powder erhältliche.

Besonders bevorzugt ist es, wenn das Siloxanelastomer in Kombination mit Ölen aus Kohlenwasserstoffen tierischer und/oder pflanzlicher Herkunft, synthetischen Ölen, synthetischen Estern, synthetischen Ethern oder deren Gemischen verwendet wird.

Ganz besonders bevorzugt ist es, wenn das Siloxanelastomer in Kombination mit unverzweigten bei Raumtemperatur flüssigen oder pastösen Silikonölen oder cyclischen Silikonölen oder deren Gemischen verwendet wird. Insbesondere vorteilhaft sind Organopolysiloxanelastomere mit der INCI-Bezeichnung Dimethicone / Polysilicone-11, ganz besonders die von der Grant Industries Inc. erhältlichen Gransil-Typen GCM, GCM-5, DMG-6, CSE Gel, PM-Gel, LTX, ININ Gel, AM-18 Gel und/oder DMCM-5.

Ganz außergewöhnlich bevorzugt ist es, wenn das Siloxanelastomer in Form eines Gels aus Siloxanelastomer und einer Lipidphase verwendet wird, wobei der Gehalt des Siloxanelastomers in dem Gel 1 bis 80 Gew.-%, bevorzugt 0,1 bis 60 Gew.-% beträgt, jeweils bezogen auf das Gesamtgewicht des Gels.

Es ist vorteilhaft im Sinne der vorliegenden Erfindung, die Gesamtmenge der Siloxanelastomere (Aktivgehalt) aus dem Bereich von 0,01 bis 10 Gew.-%, vorteilhaft von 0,1 bis 5 Gew.-%, jeweils bezogen auf das Gesamtgewicht der Formulierung, zu wählen.

Die erfindungsgemäßen kosmetischen und dermatologischen Zubereitungen können Farbstoffe und/oder Farbpigmente enthalten, insbesondere wenn sie in Form von dekorativen Kosmetika vorliegen. Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z. B. $Fe_2O_3$, $Fe_3O_4$, FeO(OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus dem Rowe Colour Index, 3. Auflage, Society of Dyers and Colourists, Bradford, England, 1971 zu wählen.

Sofern die erfindungsgemäßen Formulierungen in Form von Produkten vorliegen, welche im Gesicht angewendet werden, ist es günstig, als Farbstoff eine oder mehrere Substanzen aus der folgenden Gruppe zu wählen: 2,4-Dihydroxyazobenzol, 1-(2'-Chlor-4'-nitro-1' phenylazo)-2-hydroxynaphthalin, Ceresrot, 2-(Sulfo-1-naphthylazo)-1-naphthol-4-sulfosäure, Calciumsalz der 2-Hydroxy-1,2'-azonaphthalin-1'-sulfosäure, Calcium- und Bariumsalze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfosäure, Aluminiumsalz der 1-(4-Sulfo-1-naphthylazo)-2-naphthol-3,6-disulfosäure, 1-(4-Sulfo-1-naphthylazo)-2-naphthol-6,8-disulfosäure, Aluminiumsalz der 4-(4-Sulfo-1-phenylazo)-1-(4-sulfophenyl)-5-hydroxy-pyrazolon-3-carbonsäure, Aluminium- und Zirkoniumsalze von 4,5-Dibromfluorescein, Aluminium- und Zirkoniumsalze von 2,4,5,7-Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigo-disulfosäure, rotes und schwarzes Eisenoxid (CIN: 77 491 (rot) und 77 499 (schwarz)), Eisenoxidhydrat (CIN: 77 492), Manganammoniumdiphosphat und Titandioxid.

Ferner vorteilhaft sind öllösliche Naturfarbstoffe, wie z. B. Paprikaextrakte, β-Carotin oder Cochenille.

Vorteilhaft im Sinne der vorliegenden Erfindung sind ferner Formulierungen mit einem Gehalt an Perlglanzpigmenten. Bevorzugt sind insbesondere die im folgenden aufgelisteten Arten von Perlglanzpigmenten:

1. Natürliche Perlglanzpigmente, wie z. B.

- "Fischsilber" (Guanin/Hypoxanthin-Mischkristalle aus Fischschuppen) und
- "Perlmutt" (vermahlene Muschelschalen)

2. Monokristalline Perlglanzpigmente wie z. B. Bismuthoxychlorid (BiOCl)
3. Schicht-Substrat Pigmente: z. B. Glimmer / Metalloxid

Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere vorteihaft ist z. B. das unter der CIN 77163 aufgelistete Glanzpigment.

Vorteilhaft sind ferner beispielsweise die folgenden Perlglanzpigmentarten auf Basis von Glimmer/Metalloxid:

| Gruppe | Belegung / Schichtdicke | Farbe |
|---|---|---|
| Silberweiße Perlglanzpigmente | $TiO_2$: 40 - 60 nm | silber |
| Interferenzpigmente | $TiO_2$: 60 - 80 nm | gelb |
| | $TiO_2$: 80 - 100 nm | rot |
| | $TiO_2$: 100 - 140 nm | blau |
| | $TiO_2$: 120 - 160 nm | grün |
| Farbglanzpigmente | $Fe_2O_3$ | bronze |
| | $Fe_2O_3$ | kupfer |
| | $Fe_2O_3$ | rot |
| | $Fe_2O_3$ | rotviolett |
| | $Fe_2O_3$ | rotgrün |
| | $Fe_2O_3$ | schwarz |
| Kombinationspigmente | $TiO_2$ / $Fe_2O_3$ | Goldtöne |
| | $TiO_2$ / $Cr_2O_3$ | grün |
| | $TiO_2$ / Berliner Blau | tiefblau |

(fortgesetzt)

| Gruppe | Belegung / Schichtdicke | Farbe |
|---|---|---|
| | $TiO_2$ / Carmin | rot |

Besonders bevorzugt sind z. B. die von der Firma Merck unter den Handelsnamen Timiron, Colorona oder Dichrona erhältlichen Perlglanzpigmente.

Die Liste der genannten Perlglanzpigmente soll selbstverständlich nicht limitierend sein. Im Sinne der vorliegenden Erfindung vorteilhafte Perlglanzpigmente sind auf zahlreichen, an sich bekannten Wegen erhältlich. Beispielsweise lassen sich auch andere Substrate außer Glimmer mit weiteren Metalloxiden beschichten, wie z. B. Silica und dergleichen mehr. Vorteilhaft sind z. B. mit $TiO_2$ und $Fe_2O_3$ beschichtete $SiO_2$-Partikel ("Ronaspheren"), die von der Firma Merck vertrieben werden und sich besonders für die optische Reduktion feiner Fältchen eignen.

Es kann darüber hinaus von Vorteil sein, gänzlich auf ein Substrat wie Glimmer zu verzichten. Besonders bevorzugt sind Eisenperlglanzpigmente, welche ohne die Verwendung von Glimmer hergestellt werden. Solche Pigmente sind z. B. unter dem Handelsnamen Sicopearl Kupfer 1000 bei der Firma BASF erhältlich.

Besonders vorteilhaft sind ferner auch Effektpigmente, welche unter der Handelsbezeichnung Metasomes Standard / Glitter in verschiedenen Farben (yello, red, green, blue) von der Firma Flora Tech erhältlich sind. Die Glitterpartikel liegen hierbei in Gemischen mit verschiedenen Hilfs- und Farbstoffen (wie beispielsweise den Farbstoffen mit den Colour Index (CI) Nummern 19140, 77007, 77289, 77491) vor.

Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden. Die Gesamtmenge der Farbstoffe und farbgebenden Pigmente wird vorteilhaft aus dem Bereich von z. B. 0,1 Gew.-% bis 30 Gew.-%, vorzugsweise von 0,5 bis 15 Gew.-%, insbesondere von 1,0 bis 10 Gew.-% gewählt, jeweils bezogen auf das Gesamtgewicht der Zubereitungen.
Es ist auch vorteilhaft im Sinne der vorliegenden Erfindung, kosmetische und dermatologische Zubereitungen zu erstellen, deren hauptsächlicher Zweck nicht der Schutz vor Sonnenlicht ist, die aber dennoch einen Gehalt an weiteren UV-Schutzsubstanzen enthalten. So werden z. B. in Tagescrèmes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische und dermatologische Zubereitungen, die in der Form eines Sonnenschutzmittels vorliegen.

Dementsprechend enthalten die Zubereitungen im Sinne der vorliegenden Erfindung vorzugsweise mindestens eine weitere UV-A-, UV-B- und/oder Breitbandfiltersubstanz. Die Formulierungen können, obgleich nicht notwendig, gegebenenfalls zusätzlich zur o.g. organischen pigmentären Substanz auch ein oder mehrere organische und/oder anorganische Pigmente als UV-Filtersubstanzen enthalten, welche in der Wasser- und/oder der Ölphase vorliegen können.

Die Zubereitungen gemäß der vorliegenden Erfindung können ferner vorteilhaft auch in Form von sogenannten ölfreien kosmetischen oder dermatologischen Emulsionen vorliegen, welche eine Wasserphase und mindestens eine bei Raumtemperatur flüssige UV-Filtersubstanz als weitere Phase enthalten und welche insbesondere vorteilhaft auch frei von weiteren Ölkomponenten sein können.

Besonders vorteilhafte bei Raumtemperatur flüssige UV-Filtersubstanzen im Sinne der vorliegenden Erfindung sind Homomenthylsalicylat (INCI: Homosalate), 2-Ethylhexyl-2-cyano-3,3-diphenylacrylat (INCI: Octocrylene), 2-Ethylhexyl-2-hydroxybenzoat (2-Ethylhexylsalicylat, Octylsalicylat, INCI: Ethylhexyl Salicylate) und Ester der Zimtsäure, vorzugsweise 4-Methoxyzimtsäure(2-ethylhexyl)ester (2-Ethylhexyl-4-methoxycinnamat, INCI: Ethylhexyl Methoxycinnamate) und 4-Methoxyzimtsäureisopentylester (Isopentyl-4-methoxycinnamat, INCI: Isoamyl p-Methoxycinnamate), 3-(4-(2,2-bis Ethoxycarbonylvinyl)-phenoxy)propenyl)-methoxysiloxan/Dimethylsiloxan - Copolymer welches beispielsweise unter der Handelsbezeichnung Parsol® SLX bei Hoffmann La Roche erhältlich ist.

Bevorzugte anorganische Pigmente sind Metalloxide und/oder andere in Wasser schwerlösliche oder unlösliche Metallverbindungen, insbesondere Oxide des Titans ($TiO_2$), Zinks (ZnO), Eisens (z. B. $Fe_2O_3$), Zirkoniums ($ZrO_2$), Siliciums ($SiO_2$), Mangans (z. B. MnO), Aluminiums ($Al_2O_3$), Cers (z. B. $Ce_2O_3$), Mischoxide der entsprechenden Metalle sowie Abmischungen aus solchen Oxiden sowie das Sulfat des Bariums ($BaSO_4$).

Die Pigmente können vorteilhaft im Sinne der vorliegenden Erfindung auch in Form kommerziell erhältlicher öliger oder wäßriger Vordispersionen zur Anwendung kommen. Diesen Vordispersionen können vorteilhaft Dispergierhilfsmittel und/oder Solubilisationsvermittler zugesetzt sein.

Die Pigmente können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid ($Al_2O_3$), Aluminiumhydroxid $Al(OH)_3$, bzw. Aluminiumoxidhydrat (auch: Alumina, CAS-Nr.: 1333-84-2), Natriumhexametaphosphat $(NaPO_3)_6$, Natriummetaphosphat $(NaPO_3)_n$, Siliciumdioxid ($SiO_2$) (auch: Silica, CAS-Nr.: 7631-86-9), oder Eisenoxid ($Fe_2O_3$). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Erfindungsgemäß geeignete Zinkoxidpartikel und Vordispersionen von Zinkoxidpartikeln sind unter folgenden Handelsbezeichnungen bei den aufgeführten Firmen erhältlich:

| Handelsname | Coating | Hersteller |
|---|---|---|
| Z- Cote HP1 | 2% Dimethicone | BASF |
| Z- Cote | / | BASF |
| ZnO NDM | 5% Dimethicone | H&R |
| MZ- 303S | 3% Methicone | Tayca Corporation |
| MZ- 505S | 5% Methicone | Tayca Corporation |

Geeignete Titandioxidpartikel und Vordispersionen von Titandioxidpartikeln sind unter folgenden Handelsbezeichnungen bei den aufgeführten Firmen erhältlich:

| Handelsname | Coating | Hersteller |
|---|---|---|
| MT-100TV | Aluminiumhydroxid / Stearinsäure | Tayca Corporation |
| MT-100Z | Aluminiumhydroxid / Stearinsäure | Tayca Corporation |
| Eusolex T-2000 | Alumina / Simethicone | Merck KgaA |
| Titandioxid T805 (Uvinul $TiO_2$) | Octyltrimethylsilan | Degussa |
| Tioveil AQ 10PG | Alumina / Silica | Solaveil /Uniquema |
| Eusolex T-aqua | Wasser/Alumina/Natriummetaphosphat | Merck |

Weitere vorteilhafte Pigmente sind Latexpartikel. Erfindungsgemäß vorteilhafte Latexpartikel sind die in den folgenden Schriften beschriebenen: US 5,663,213 bzw. EP 0 761 201. Besonders vorteilhafte Latexpartikel sind solche, welche aus Wasser und Styrol/Acrylat-Copolymeren gebildet werden und z. B. unter der Handelsbezeichnung "Alliance SunSphere" bei der Fa. Rohm & Haas erhältlich sind.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Vorteilhafte weitere UV-Filtersubstanzen im Sinne der vorliegenden Erfindung sind sulfonierte, wasserlösliche UV-Filter, wie z. B.:

- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Disodium Phenyl Dibenzimidazol Tetrasulfonat (CAS-Nr.: 180898-37-7), welches beispielsweise unter der Handelsbezeichnung Neo Heliopan AP bei Haarmann & Reimer erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Haarmann

& Reimer erhältlich ist;

- 1,4-di(2-oxo-10-Sulfo-3-bornylidenmethyl)-Benzol (auch: 3,3'-(1,4-Phenylendimethylene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird. Benzol-1,4-di(2-oxo-3-bornylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;
- Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Vorteilhafte UV-Filtersubstanzen im Sinne der vorliegenden Erfindung sind ferner Benzoxazol-Derivate, welche sich durch die folgende Strukturformel auszeichnen,

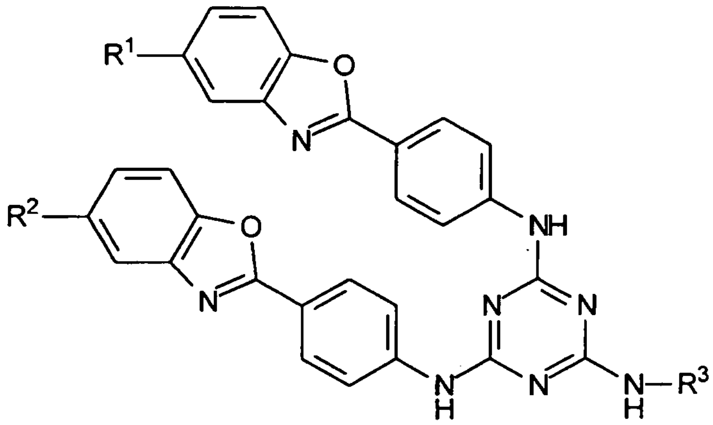

worin $R^1$, $R^2$ und $R^3$ unabhängig voneinander gewählt werden aus der Gruppe der verzweigten oder unverzweigten, gesättigten oder ungesättigten Alkylreste mit 1 bis 10 Kohlenstoffatomen. Es ist erfindungsgemäß besonders vorteilhaft, die Reste $R^1$ und $R^2$ gleich zu wählen, insbesondere aus der Gruppe der verzweigten Alkylreste mit 3 bis 5 Kohlenstoffatomen. Es ist ferner besonders vorteilhaft im Sinne der vorliegenden Erfindung, wenn $R^3$ einen unverzweigten oder verzweigten Alkylrest mit 8 Kohlenstoffatomen, insbesondere den 2-Ethylhexylrest darstellt.

Erfindungsgemäß besonders bevorzugtes Benzoxazol-Derivat ist das 2,4-bis-[5-1(dimethylpropyl)benzoxazol-2-yl-(4-phenyl)-imino]-6-(2-ethylhexyl)-imino-1,3,5-triazin mit der CAS Nr. 288254-16-0, welches sich durch die Strukturformel

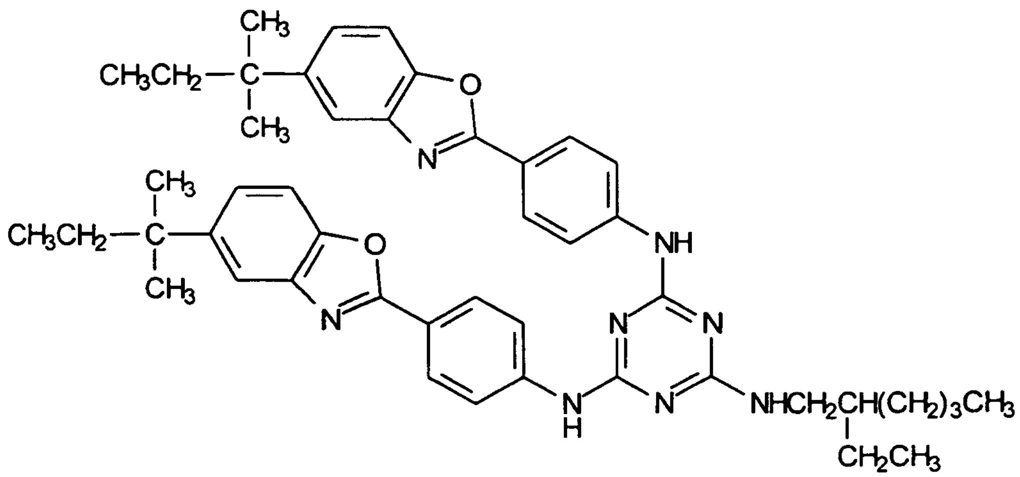

auszeichnet und bei 3V Sigma unter der Handelsbezeichnung Uvasorb® K2A erhältlich ist.

Das oder die Benzoxazol-Derivate liegen vorteilhaft in gelöster Form in den erfindungsgemäßen kosmetischen

Zubereitungen vor. Es kann ggf. aber auch von Vorteil sein, wenn das oder die Benzoxazol-Derivate in pigmentärer, d. h. ungelöster Form - beispielsweise in Partikelgrößen von 10 nm bis zu 300 nm - vorliegen.

Vorteilhafte UV-Filtersubstanzen im Sinne der vorliegenden Erfindung sind ferner sogenannte Hydroxybenzophenone. Hydroxybenzophenone zeichnen sich durch die folgende Strukturformel aus:

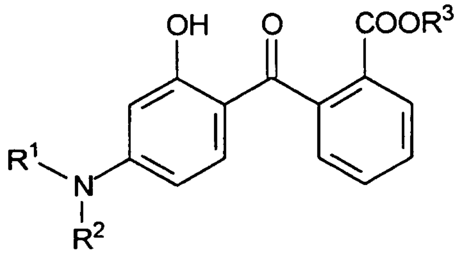

worin

- $R^1$ und $R^2$ unabhängig voneinander Wasserstoff, $C_1$-$C_{20}$-Alkyl, $C_3$-$C_{10}$-Cycloalkyl oder $C_3$-$C_{10}$-Cycloalkenyl bedeuten, wobei die Substituenten $R^1$ und $R^2$ gemeinsam mit dem Stickstoffatom, an das sie gebunden sind, einen 5- oder 6-Ring bilden können und
- $R^3$ einen $C_1$-$C_{20}$-Alkyl Rest bedeutet.

Ein besonders vorteilhaftes Hydroxybenzophenon im Sinne der vorliegenden Erfindung ist der 2-(4'-Diethylamino-2'-hydoxybenzoyl)-benzoesäurehexylester (auch: Aminobenzophenon), welcher sich durch folgende Struktur auszeichnet:

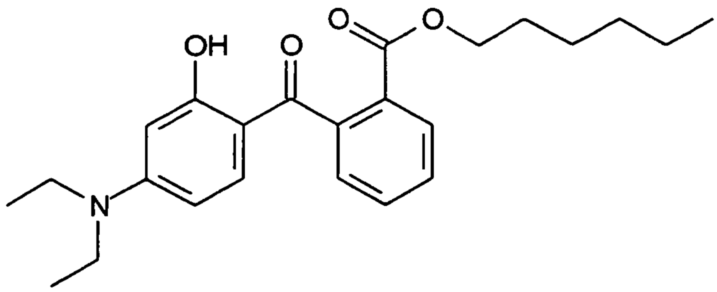

und unter dem Uvinul A Plus bei der Fa. BASF erhältlich ist.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Triazinderivate, wie z. B.

- 2,4-Bis-{[4-(2-Ethyl-hexyloxy)-2-hydroxy]-phenyl}-6-(4-methoxyphenyl)-1,3,5-triazin (INCI: Bis-Ethylhexyloxylphenol Methoxyphenyl Triazin), welches unter der Handelsbezeichnung Tinosorb® S bei der CIBA-Chemikalien GmbH erhältlich ist;
- Dioctylbutylamidotriazon (INCI: Diethylhexyl Butamido Triazone), welches unter der Handelsbezeichnung UVASORB HEB bei Sigma 3V erhältlich ist;
- 4,4',4"-(1,3,5-Triazin-2,4,6-triyltriimino)-tris-benzoësäure-tris(2-ethylhexylester), auch: 2,4,6-Tris-[anilino-(p-carbo-2'-ethyl-1'-hexyloxy)]-1,3,5-triazin (INCI: Ethylhexyl Triazone), welches von der BASF Aktiengesellschaft unter der Warenbezeichnung UVI-NUL® T 150 vertrieben wird;
- 2-[4,6-Bis(2,4-dimethylphenyl)-1,3,5-triazin-2-yl]-5-(octyloxy)phenol (CAS Nr.: 2725-22-6).

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane.

Die weiteren UV-Filtersubstanzen können öllöslich oder wasserlöslich sein. Vorteilhafte öllösliche Filtersubstanzen sind z. B.:

- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- 2,4,6-Trianilino-(p-carbo-2'-ethyl-1'-hexyloxy)-1,3,5-triazin;
- Ester der Benzalmalonsäure, vorzugsweise 4-Methoxybenzalmalonsäuredi(2-ethylhexyl)ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon sowie
- an Polymere gebundene UV-Filter.

Vorteilhafte wasserlösliche Filtersubstanzen sind z. B.:

Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 T erhältlich ist.

Besonders vorteilhafte Zubereitungen im Sinne der vorliegenden Erfindung, die sich durch einen hohen bzw. sehr hohen UV-A-Schutz auszeichnen, enthalten neben der oder den erfindungsgemäßen Filtersubstanz(en) bevorzugt ferner weitere UV-A- und/oder Breitbandfilter, insbesondere Dibenzoylmethanderivate [beispielsweise das 4-(tert.-Butyl)-4'-methoxydibenzoylmethan] und/oder das 2,4-Bis-{[4-(2-Ethyl-hexyloxy)-2-hydroxy]-phenyl}-6-(4-methoxyphenyl)-1,3,5-triazin und/oder Phenylen-1,4-bis-(2-benzimidazyl)-3,3'-5,5'-tetrasulfonsäure-bis-natriumsalz jeweils einzeln oder in beliebigen Kombinationen miteinander.

Die Liste der genannten UV-Filter, die im Sinne der vorliegenden Erfindung eingesetzt werden können, soll selbstverständlich nicht limitierend sein.

Vorteilhaft enthalten die Zubereitungen gemäß der vorliegenden Erfindung die Substanzen, die UV-Strahlung im UV-A- und/oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

**Beispiele:**

**1. PIT - Emulsionen (Spray)**

| | 1 | 2 | 3 | 4 | 5 | 6 | 7 | 8 |
|---|---|---|---|---|---|---|---|---|
| Glycerinmonostearat SE | 0,50 | 2,00 | 3,00 | 5,00 | | | 0,50 | 4,00 |
| Glyceryllsostearat | | | | | 3,50 | 4,00 | 2,00 | |
| Isoceteth-20 | | 0,50 | | | 2,00 | | | |
| Ceteareth-12 | | 5,00 | | 1,00 | | | | 3,50 |
| Ceteareth-20 | | | | 2,00 | | 2,50 | 3,00 | |
| PEG-100 Stearat | 5,00 | | 1,00 | | 1,00 | | | 0,50 |
| Cetyl Alkohol | 2,50 | 1,00 | | 1,50 | | 0,50 | 1,50 | |
| Cetyl Palmitat | | | | 0,50 | | 1,00 | | |
| Cetyl Dimethicon Copolyol | 0,50 | | | | 0,50 | | 1,00 | |
| Polyglyceryl-2 Dipolyhydroxystearat | | | | 0,75 | 0,25 | | | |
| Aniso Triazin | | | 0,50 | 2,00 | | 3,00 | | |
| Butyl Methoxydibenzoylmethan | 1,50 | | 1,00 | | 5,00 | | | |

(fortgesetzt)

| | 1 | 2 | 3 | 4 | 5 | 6 | 7 | 8 |
|---|---|---|---|---|---|---|---|---|
| Bisimidazylat | | 2,00 | | | 1,00 | | | |
| Terephthaliden Dicampher Sulfonsäure | | | 0,50 | | | | 1,00 | |
| Drometrizol Trisiloxan | | | 2,00 | | | 3,00 | | 1,00 |
| Ethylhexyl Methoxycinnamat | 8,00 | | | 4,50 | 5,00 | 8,00 | | |
| Ethylhexyl Salicylat | 4,00 | | | | 3,50 | 4,00 | | |
| Dioctyl Butamidotriazon | | | | 3,00 | 2,00 | 2,00 | | 1,50 |
| Ethylhexyl Triazon | | | 2,00 | 4,00 | | | 1,50 | 3,00 |
| Dimethicon Diethylbenzalmalonat | | | | | | | | |
| 4-Methylbenzyliden Campher | | | | | | | 3,00 | |
| Octocrylen | | | 5,00 | | 8,00 | | | 7,50 |
| Phenylbenzimidazol Sulfonsäure | 1,00 | 5,00 | | 3,00 | 1,00 | | | |
| Decylglucosid | 0,01 | 0,10 | 1,00 | 0,20 | 0,20 | 0,20 | 0,50 | 0,10 |
| Methylen Bis-Benzotriazolyl Tetramethylbutylphenol | 1,00 | 5,00 | 10,0 | 3,00 | 1,00 | 3,00 | 3,00 | 0,50 |
| Pentanatrium-Ethylendiamintetramethylenphosphonat | 0,05 | 0,10 | 1,00 | 0,01 | 0,10 | 0,50 | 0,50 | 0,10 |
| Al-Stearat + $TiO_2$ | | | | | | | | 1,50 |
| C12-15 Alkyl Benzoat | | | | | | 4,00 | | |
| Cocoglyceride | | 3,00 | | 3,00 | | 2,50 | | 3,50 |
| Dicaprylyl Ether | 4,00 | | | | | | | |
| Butylenglycol Dicaprylat/Dicaprat | | 4,00 | | 3,00 | | | | |
| Dicaprylyl Carbonat | | | | 0,50 | | | | 6,00 |
| Dibutyl Adipate | | | 2,50 | | | 3,00 | | 1,00 |
| Phenyltrimethicon | 2,00 | | | | | 3,00 | | |
| Cyclomethicon | | 3,00 | | | | | | 4,00 |
| PVP Hexadecen Copolymer | | | | 1,00 | 1,50 | | | |
| Glycerin | 10,0 | 5,00 | | 7,50 | | | | |
| Tocopherol | 1,00 | | | 0,75 | 0,50 | | 1,00 | |
| Shea Butter | | 2,00 | | | | | | 0,50 |
| Iodopropyl Butylcarbamat | 0,12 | | | | 0,20 | 0,15 | | |
| DMDM Hydantoin | | | | 0,10 | | | | |
| Methylparaben | | 0,50 | 0,25 | | 0,45 | | | |
| Phenoxyethanol | 0,50 | 0,40 | | 1,00 | | | | 1,00 |
| Octoxyglycerin | | 0,30 | | | 1,00 | | | |
| Ethanol | | | | 2,00 | | | 7,50 | 4,00 |
| Parfüm | 0,20 | | 0,20 | 0,20 | 0,45 | | | 0,20 |
| Wasser | ad. 100 | ad. 100 | ad. 100 | ad. 100 | ad. 100 | ad. 100 | ad. 100 | ad. 100 |

**2. Hydrodispersions- und O/W - Sprays**

| | 1 | 2 | 3 | 5 | 6 | 7 | 8 | 9 |
|---|---|---|---|---|---|---|---|---|
| Sodium Carbomer | 0,7 | 0,2 | | 0,4 | | | 1,5 | |
| Acrylates/C10-30 Alky Acrylate Crosspolymer | | 0,5 | 0,3 | 0,2 | 1,0 | | | 1,5 |
| Hydroxypropyl Cellulose | | 0,5 | | 1,0 | | 0,5 | | |
| Ceteareth-20 | 1,0 | | | | 2,0 | | | |
| PEG-40 Stearat | | | 2,0 | | | 1,0 | | |
| Cetyl Alkohol | | | 0,5 | | 1,0 | | | |
| Hydrogenated Cocoglyceride | | | | | 0,5 | 1,0 | | |
| Polyglyceryl-2 Dipolyhydroxystearat | 0,2 | | | | | 0,5 | | |
| Decylglucosid | 0,01 | 0,10 | 1,00 | 0,20 | 0,20 | 0,20 | 0,50 | 0,10 |
| Methylen Bis-Benzotriazolyl Tetramethylbutylphenol | 1,00 | 5,00 | 10,0 | 3,00 | 1,00 | 3,00 | 3,00 | 0,50 |
| Pentanatrium-Ethylendiamintetramethylenphosphonat | 0,05 | 0,10 | 1,00 | 0,01 | 0,10 | 0,50 | 0,50 | 0,10 |
| Aniso Triazin | | 2,0 | | 3,0 | 2,0 | | 0,5 | |
| Butyl Methoxydibenzoylmethan | | | 0,5 | | 1,0 | | 0,5 | |
| Bisimidazylat | 0,5 | | | 0,5 | | 3,0 | 0,5 | |
| Terephthaliden Dicampher Sulfonsäure | 0,5 | | | | 0,5 | | 0,5 | |
| Drometrizol Trisiloxan | 3,0 | | | 1,0 | | | | 4,0 |
| Ethylhexyl Methoxycinnamat | | 10,0 | | 7,5 | | | | 5,0 |
| Ethylhexyl Salicylat | | 5,0 | | 0,5 | | 1,0 | | |
| Homosalat | | 4,0 | | | | | | |
| Octocrylen | 6,0 | 4,0 | 2,0 | 7,5 | | | | 10,0 |
| Dimethicon Diethylbenzalmalonat | | | 4,0 | | | 6,0 | | |
| Dioctyl Butamidotriazon | 1,0 | 2,5 | | | | | 3,5 | |
| Ethylhexyl Triazon | | | 3,0 | 2,0 | | 1,5 | | 4,0 |
| 4-Methylbenzyliden Campher | 2,0 | | | | | 2,0 | | |
| Phenylbenzmidazol Sulfonsäure | | 1,0 | | 2,0 | | | | |
| Titandioxid | 2,0 | | | 3,0 | | 0,5 | 4,0 | 1,5 |
| Zinkoxid | | | | | 3,0 | 1,0 | | |
| C12-15 Alkyl Benzoat | 5,0 | | | | 5,0 | | | |
| Cocoglyceride | 2,5 | | | | | | 8,0 | 4,0 |
| Dicaprylyl Ether | | | | | 5,0 | | | |
| Butylenglycol Dicaprylat/Dicaprat | | | 7,5 | | | | | 6,0 |
| Dicaprylyl Carbonat | | | 4,0 | | | | 10,0 | |
| Paraffinöl | | | | | | 7,0 | | |
| Dimethicon | 5,0 | | 2,5 | | 1,0 | | | |

(fortgesetzt)

| | 1 | 2 | 3 | 5 | 6 | 7 | 8 | 9 |
|---|---|---|---|---|---|---|---|---|
| PVP Hexadecen Copolymer | | 0,75 | | | 0,5 | 1,0 | 1,0 | |
| Glycerin | 0,3 | 10,0 | 7,5 | 5,0 | | 5,0 | | 7,5 |
| Vitamin E -Acetat | | 0,5 | 0,75 | | 1,0 | | 1,5 | |
| Ascorbyl - Palmitat | 0,5 | | 0,25 | | | | | 0,75 |
| Vaseline | | | 2,0 | | | 1,0 | | |
| DMDM Hydantoin | | 0,12 | | | | | | 0,2 |
| Iodopropyl Butylcarbamat | | | | | 0,18 | | 0,2 | |
| Glycin Soja | 1,5 | | | 2,0 | | | | |
| Propylparaben | | 0,02 | | | | | 0,1 | |
| Phenoxyethanol | 1,0 | | 1,0 | | 0,75 | | | |
| Ethanol | | | 3,0 | 7,5 | | 5,0 | | 3,5 |
| Trisodium EDTA | 0,1 | 0,3 | | 0,5 | | | 0,2 | 0,15 |
| Farbstoff wasserlöslich | | | 0,02 | | | 0,10 | | |
| Parfüm | 0,4 | | 0,2 | | 0,3 | 0,3 | 0,1 | |
| Wasser | ad. 100 | ad. 100 | ad. 100 | ad. 100 | ad. 100 | ad. 100 | ad. 100 | ad. 100 |

**3. W/O-Emulsion**

| | 1 | 2 | 3 | 4 | 5 | 6 |
|---|---|---|---|---|---|---|
| Polyglyceryl-3 Diisostearat | 6,0 | | | | | |
| Polyglyceryl-2 Dipolyhydroxystearat | | 5,0 | | 6,0 | | 3,0 |
| PEG-30 Dipolyhydroxystearat | | | 5,5 | | | |
| Cetyl Dimethicon Copolyol | | | | | 1,5 | 4,0 |
| Laurylmethicon Copolyol | | | | | 4,0 | |
| Aminobenzophenon | | | | 5,0 | | |
| Aniso Triazin | | 2,0 | 0,5 | | | |
| Dioctyl Butamidotriazon | | 1,0 | 2,5 | | 2,0 | |
| Ethylhexyl Triazon | | 2,0 | | | | |
| Bisoctyltriazol | 1,5 | | | 4,0 | | |
| Drometrizol Trisiloxan | 2,0 | | 3,0 | | | |
| Phenylbenzmidazole Sulfonsäure | | 1,0 | | | | |
| Bisimidazylate | | | | 2,5 | 2,0 | 2,0 |
| Terephthalylidene Dicamphor Sulfonic Acid | 0,75 | | | | | |
| Ethylhexyl Methoxycinnamat | | 7,5 | 5,0 | | | |
| Octocrylen | | | 5,0 | | 10,0 | |
| Dimethicone-diethylbenzalmalonat | 7,0 | | | | | |

(fortgesetzt)

| | 1 | 2 | 3 | 4 | 5 | 6 |
|---|---|---|---|---|---|---|
| Ethylhexyl Salicylate | | | 5,0 | | | |
| Homosalate | | | 3,5 | | | |
| Butyl Methoxydibenzoylmethan | 1,5 | | | | | |
| 4-Methylbenzylidene Campher | 3,0 | | | | | |
| Methylen Bis-Benzotriazolyl Tetramethylbutylphenol | 1,00 | 5,00 | 10,0 | 3,00 | 3,00 | 3,00 |
| Decylglucosid | 0,01 | 0,10 | 1,00 | 0,20 | 0,20 | 0,50 |
| Pentanatrium-Ethylendiamintetramethylenphosphonat | 0,05 | 0,10 | 1,00 | 0,01 | 0,50 | 0,50 |
| Mikronisiertes Titandioxid | | 3,0 | 6,0 | | 2,0 | 2,0 |
| Mikronisiertes Zinkoxid | | | | | 7,0 | |
| Paraffinöl | 20,0 | 15,0 | | 10,0 | | 15,0 |
| Vaseline | | 2,0 | | 5,0 | | |
| Cyclomethicon | | | | | | 10,0 |
| Dimethicon | | | 4,0 | | 3,0 | |
| Dicaprylylcarbonat | 10,0 | | 9,0 | | | 10,0 |
| $C_{12-15}$ Alkyl Benzoat | 5,0 | | | 10,0 | 9,0 | |
| Butylen Glycol Dicaprylat/Dicaprat | | 10,0 | | | 10,0 | |
| Octyldodecanol | | | 10,0 | 15,0 | | 5,0 |
| Magnesiumsulfat | 0,7 | 0,5 | | 0,4 | 1,0 | |
| Glycerin | | 10,0 | 5,0 | 7,5 | | 3,0 |
| Parfum | 0,45 | 0,2 | 0,3 | | 0,4 | |
| Ethanol | | 3,5 | | | | 5,0 |
| Octoxyglycerin | | | 0,5 | | | 1,0 |
| Tocopherol oder Tocopherolacetat | 0,5 | 0,75 | | 0,3 | 0,5 | |
| Trisodium EDTA | 0,1 | 0,2 | | 0,1 | 0,3 | |
| Phenonip® | | 0,4 | 0,5 | | 0,5 | |
| DMDM Hydantoin | 0,1 | | | 0,2 | | |
| Wasser | ad 100 | ad 100 | ad 100 | ad 100 | ad 100 | ad 100 |

**4. W/O - Sonnenschutzemulsionen**

| | 7 | 8 | 9 | 10 | 11 | 12 |
|---|---|---|---|---|---|---|
| PEG-7 Hydrogenated Castor Oil | 5,0 | | | | | |
| Polyglyceryl-2 Dipolyhydroxystearat | | | | 6,0 | 4,0 | 2,0 |
| PEG-30 Dipolyhydroxystearat | | 5,0 | | | | 1,0 |
| Cetyl Dimethicon Copolyol | | | 0,5 | | | 1,0 |
| Polyglyceryl Polyricinoleat | | | 5,0 | | 1,5 | |

(fortgesetzt)

| | 7 | 8 | 9 | 10 | 11 | 12 |
|---|---|---|---|---|---|---|
| Aminobenzophenon | | | 5,0 | | | 2,5 |
| Aniso Triazin | 2,0 | 2,0 | | 1,0 | 0,5 | 3,0 |
| Dioctyl Butamidotriazon | | 2,0 | | 1,0 | 3,0 | |
| Ethylhexyl Triazon | | 2,0 | | 4,0 | | |
| Bisoctyltriazol | 4,0 | | | | | 4,0 |
| Drometrizol Trisiloxan | | 3,0 | | | | 4,0 |
| Phenylbenzmidazole Sulfonsäure | | | 2,5 | 0,5 | 2,0 | 1,0 |
| Bisimidazylate | 2,0 | | | 1,5 | | |
| Terephthalylidene Dicamphor Sulfonic Acid | | 0,5 | | | | |
| Ethylhexyl Methoxycinnamat | | | | 10,0 | | |
| Octocrylen | | | 5,0 | | | 7,5 |
| Dimethicone-diethylbenzalmalonat | | | 4,0 | | | |
| Ethylhexyl Salicylate | | | | | 3,0 | |
| Homosalate | | | 4,0 | | | 5,0 |
| Butyl Methoxydibenzoylmethan | | | 3,0 | | 1,0 | 2,5 |
| Methylen Bis-Benzotriazolyl Tetramethylbutylphenol | 1,00 | 10,0 | 3,00 | 1,00 | 3,00 | 0,50 |
| Decylglucosid | 0,01 | 1,00 | 0,20 | 0,20 | 0,50 | 0,10 |
| Pentanatrium-Ethylendiamintetramethylenphosphonat | 0,05 | 1,00 | 0,01 | 0,10 | 0,50 | 0,10 |
| Mikronisiertes Titandioxid | 5,0 | | | 3,0 | | |
| Mikronisiertes Zinkoxid | | | 5,0 | 3,0 | 8,0 | |
| Isohexadecen | | 10,0 | | 5,0 | | 15,0 |
| Coco-Caprylat/Caprat | 6,0 | 5,0 | 10,0 | | 4,5 | |
| Cetyl Dimethicon | | | | 1,0 | | 0,75 |
| Dimethicon | | | 2,5 | 4,0 | 5,5 | |
| Polydecen | | 10,0 | 7,0 | | | |
| $C_{12-15}$ Alkyl Benzoat | 9,0 | 4,0 | | 10,0 | | |
| Polyisobuten | 0,5 | | 2,0 | | | 1,0 |
| Natriumchlorid | | | 0,45 | 0,55 | 0,6 | 1,5 |
| Butylen Glycol | 10,0 | 7,5 | | 15,0 | | 5,0 |
| Parfum | 0,4 | | 0,15 | | 0,5 | |
| Glycin Soja | | | 2,0 | 1,0 | | 1,0 |
| Ethanol | | | | 5,0 | | 5,0 |
| Tocopherol oder Tocopherolacetat | 0,5 | 0,75 | | 0,3 | 1,0 | |
| Trisodium EDTA | 0,2 | 0,4 | | | | 0,2 |
| Phenoxyethanol | 0,5 | | 1,0 | | 1,0 | |
| DMDM Hydantoin | 0,05 | 0,1 | | | | 0,2 |
| Wasser | ad 100 | ad 100 | ad 100 | ad 100 | ad 100 | ad 100 |

**5. O/W-Emulsionen**

| | 1 | 2 | 3 | 4 | 5 | 6 |
|---|---|---|---|---|---|---|
| Glycerylstearat Citrat | 2.00 | 1.50 | 3.00 | | | |
| Glycerylstearat SE | | | | | 2.00 | |
| Glycerylstearat | | | | 2.00 | | 2.00 |
| PEG-40 Stearat | | | | | | 2.00 |
| Stearinsäure | | | | 3.00 | 2.00 | |
| Caprylic/Capric Triglycerid | 0.50 | | 1.00 | | | 1.50 |
| Cetearyl Alkohol | 3.00 | 1.00 | | | 2.00 | |
| Cetyl Alkohol | | | 3.00 | | | 2.00 |
| Stearyl Alkohol | | 1.00 | | | 0.50 | 2.00 |
| Lanolin Alkohol | | 0.50 | | 0.50 | 0.50 | |
| Myristyl Alkohol | | | | 3.00 | 2.00 | |
| Octyldodecanol | 1.50 | | | | | |
| Mineralöl | | | 2.00 | | | |
| Methylen Bis-Benzotriazolyl Tetramethylbutylphenol | 1,00 | 10,0 | 3,00 | 1,00 | 3,00 | 3,00 |
| Decylglucosid | 0,01 | 1,00 | 0,20 | 0,20 | 0,20 | 0,50 |
| Pentanatrium-Ethylendiamintetramethylenphosphonat | 0,05 | 1,00 | 0,01 | 0,10 | 0,50 | 0,50 |
| Dimethicodiethylbenzalmalonat | | | | 5,00 | | |
| Ethylhexyl Methoxycinnamat | 6,50 | 5,00 | | | | 10,00 |
| Bis-Ethylhexyloxyphenol methoxyphenyl Triazin | 2,50 | 2,00 | 1,50 | | | 1,00 |
| Butyl Methoxydibenzoylmethan | 1,10 | | 1,00 | | 2,00 | |
| Phenyl Dibenzimidazol Tetrasulfonsäure | | | 0,50 | 2,00 | | |
| Ethylhexyl Triazon | 2,20 | | | | | |
| 4-Methylbenzyliden Campher | | | | | 2,00 | |
| Octocrylen | | 5,00 | | | | 3,50 |
| Diethylhexyl Butamido Triazon | 1,00 | 3,00 | | | 1,50 | |
| Phenylbenzmidazol Sulfonsäure | | | | 1,00 | | 2,00 |
| Drometrizol Trisiloxan | | | | | | 2,00 |
| Terephthaliden Dicamphor Sulfonsäure | | | | | | 0,50 |
| Benzophenon-3 | | | | 2,00 | | |
| Ethylhexyl Salicylat | | | | | 5,00 | |
| Homosalat | | | | 3,00 | | |
| Isoamyl p-Methoxycinnamat | | | | | 1,00 | |
| Titandioxid Aluminium-hydroxid/Stearinsäure coating | 2,00 | 1,50 | 3,00 | | | 1,00 |
| Zinkoxid HP1 | | | 1,00 | | | |
| C12-C15 Alkyl Benzoat | 2.00 | | 1.50 | 2.50 | | 0.80 |

(fortgesetzt)

| | 1 | 2 | 3 | 4 | 5 | 6 |
|---|---|---|---|---|---|---|
| Dicaprylyl Carbonat | | 3.00 | | | 2.00 | |
| Butylenglycol Dicaprylat/Dicaprat | 5.00 | | | 6.50 | 4.50 | 3.00 |
| Butylenglycol | | 7.50 | | 4.50 | | 5.00 |
| Carbomer | 0.30 | 0.10 | 0.20 | | 0.30 | 0.35 |
| Dicaprylyl Ether | 2.50 | 0.50 | | 2.00 | | 1.50 |
| PVP/Hexadecen Copolymer | 1.00 | | 0.80 | 1.00 | | 0,50 |
| Xanthan Gum | 0.20 | | 0.40 | | 0.60 | 0.30 |
| Dimethicon | 2.00 | | | 1.00 | | |
| Cyclomethicon | | 2.50 | | | | |
| Tocopheryl Acetat | 1.00 | | | | | 0.50 |
| Glycerin | 5.00 | | 7.50 | | 3.50 | 2.00 |
| Farbstoff (wasser- und/oder öllöslich) | | | 0.25 | | | 0.15 |
| DMDM Hydantoin | | | 0.20 | | | 0.40 |
| Propylparaben | | 0.40 | | 0.25 | | |
| Methylparaben | 0.60 | | | 0.30 | | |
| Ethanol | | | | | 1.50 | |
| Konkaben LMB ® | | | | | 0.20 | |
| Trinatrium EDTA | 1.00 | | | | | 1.00 |
| Phenoxyethanol | 0.15 | | 0.30 | | 0.50 | 0.40 |
| Parfüm | q.s. | q.s. | q.s. | q.s. | q.s. | q.s. |
| Aqua | Ad 100 | Ad 100 | Ad 100 | Ad 100 | Ad 100 | Ad 100 |

**6. O/W-Emulsionen**

| | 7 | 8 | 9 | 10 | 11 | 12 | 13 |
|---|---|---|---|---|---|---|---|
| Polyglyceryl 3-Methylglucose Distearat | 3.00 | | | | | | |
| Glycerylstearat Citrat | | | | | | | 2.50 |
| Glycerylstearat SE | | | 3.00 | 2.00 | | 3.00 | |
| PEG-40 Stearat | | | | 1.00 | | | |
| PEG-100 Stearat | | | | | | 1.00 | |
| Sorbitan Stearat | 1.00 | | | | | | |
| Natrium Cetearyl Sulfat | | | 1.00 | | | | 0.50 |
| Cetearylpolyglucosid | | 2.00 | | | | | |
| Caprylic/Capric Triglycerid | | | 1.50 | | | | 1.00 |
| Cetearyl Alkohol | | | | 2.00 | | | |
| Cetyl Alkohol | 1.00 | | | | | 1.00 | |
| Stearyl Alkohol | | 5.00 | | | | | |

(fortgesetzt)

| | 7 | 8 | 9 | 10 | 11 | 12 | 13 |
|---|---|---|---|---|---|---|---|
| Behenyl Alkohol | 1.00 | | | | | | |
| Octyldodecanol | | | | | | | 2.00 |
| Mineralöl | | | | | | | 1.50 |
| Ethylhexyl Methoxycinnamat | | 8,00 | | | 3.00 | | 4,00 |
| Bis-Ethylhexyloxyphenol methoxyphenyl Triazin | 2,00 | | 1,50 | | 2.00 | | 2,00 |
| Butyl Methoxydibenzoylmethan | | 3,00 | | | | | 1,00 |
| Phenyl Dibenzimidazol Tetrasulfonsäure | | | 1,50 | | | | 0,50 |
| Ethylhexyl Triazon | 2,50 | | | 4,00 | | | 2,00 |
| 4-Methylbenzyliden Camphor | | 2,00 | | | | | |
| Octocrylen | | 5,00 | | | | | |
| Diethylhexyl Butamido Triazon | | | 2,50 | | 1,50 | | 1,00 |
| Phenylbenzmidazol Sulfonsäure | | | 2,00 | 1,00 | | | |
| Methylen Bis-Benzotriazolyl Tetramethylbutylphenol | 1,00 | 10,0 | 3,00 | 1,00 | 1,00 | 3,00 | 0,50 |
| Decylglucosid | 0,01 | 1,00 | 0,20 | 0,20 | 0,10 | 0,50 | 0,10 |
| Pentanatrium-Ethylendiamintetramethylenphosphonat | 0,05 | 1,00 | 0,01 | 0,10 | 0,50 | 0,50 | 0,10 |
| Ethylhexyl Salicylat | | | | | 4,00 | | |
| Homosalat | 4,50 | | | | | | |
| Titandioxid Silikon coating | | | 3,00 | 2,00 | 1,00 | | 2,00 |
| Zinkoxid HP-1 | 2,00 | | | 3,00 | | | |
| C12-C15 Alkyl Benzoat | 2.00 | | 1.50 | 2.50 | | | 0.80 |
| Dicaprylyl Carbonat | | 3.00 | | | | 2:00 | |
| Butylenglycol Dicaprylat/Dicaprat | 5.00 | | | 6.50 | | 4.50 | 3.00 |
| Butylenglycol | | 7.50 | | 4.50 | 9.00 | | 5.00 |
| Carbomer | 0.30 | 0.10 | 0.20 | | | 0.30 | 0.35 |
| Tocopheryl Acetat | 0.30 | | 0.50 | | | 0.50 | 0.80 |
| Dicaprylyl Ether | 2.50 | 0.50 | | 2.00 | | | 1.50 |
| PVP/Hexadecen Copolymer | 1.00 | | 0.80 | 1.00 | | | 0,50 |
| Xanthan Gum | 0.20 | | 0.40 | | 1,00 | 0.60 | 0.30 |
| Dimethicon | 2.00 | | | 1.00 | | | |
| Cyclomethicon | | 2.50 | | | 5.00 | | |
| Glycerin | 5.00 | | 7.50 | | 4,00 | 3.50 | 2.00 |
| Farbstoff (wasser- und/oder öllöslich) | 0.15 | 0.25 | | 0.20 | | | 0.10 |
| DMDM Hydantoin | | | 0.20 | | | | 0.40 |
| Parabene | 0.60 | | | 0.30 | | | |
| Ethanol | | | | | 5,00 | 1.50 | |

(fortgesetzt)

| | 7 | 8 | 9 | 10 | 11 | 12 | 13 |
|---|---|---|---|---|---|---|---|
| Konkaben LMB ® | | | | | | 0.20 | |
| Trinatrium EDTA | 1.00 | | | | 0,50 | | |
| Phenoxyethanol | 0.15 | | 0.30 | | | 0.50 | 0.40 |
| Parfüm | q.s. | q.s. | q.s. | q.s. | q.s. | q.s. | q.s. |
| Aqua | Ad 100 | Ad 100 | Ad 100 | Ad 100 | Ad 100 | Ad 100 | Ad 100 |

**7. schäumende Emulsionen**

| | 1 | 2 | 3 | 4 |
|---|---|---|---|---|
| Stearinsäure | 2,00 | | | |
| Cetylalkohol | 2,00 | | | |
| PEG-40 Stearat | 1.50 | | | |
| Sorbitan Stearat | 0,50 | | | |
| Hydrogenated Polyisobuten | 2,50 | | | |
| Beheneth-9 | | 4,50 | | |
| Dicaprylyl Ether | | 8,00 | | 7,00 |
| Butylenglycol Dicaprylat/Caprat | | | | 4,00 |
| Cetearyl Isononanoat | | 4,00 | | |
| Glyceryl Stearat | | | 2,00 | |
| Octyl Palmitate | | | 8,00 | |
| Ceteareth 20 | | | 1,50 | 2,00 |
| Ceteareth 12 | | | 3,00 | |
| Ethylhexyl Methoxycinnamat | | 4,00 | | 6,80 |
| Bis-Ethylhexyloxyphenol methoxyphenyl Triazin | 2,60 | | 2,00 | |
| Butyl Methoxydibenzoylmethan | | 2,00 | | |
| Phenyl Dibenzimidazol Tetrasulfonsäure | | | 1,00 | 1,50 |
| Ethylhexyl Triazon | 1,80 | 2,50 | | |
| 4-Methylbenzyliden Camphor | | 3,00 | | |
| Octocrylen | | | 6,00 | 8,50 |
| Diethylhexyl Butamido Triazon | 1,50 | | | 3,00 |
| Phenylbenzmidazol Sulfonsäure | | | | 1,00 |
| Methylen Bis-Benzotriazolyl Tetramethylbutylphenol | 1,00 | 10,0 | 3,00 | 1,00 |
| Decylglucosid | 0,01 | 1,00 | 0,20 | 0,20 |
| Pentanatrium-Ethylendiamintetramethylenphosphonat | 0,05 | 1,00 | 0,01 | 0,10 |
| Ethylhexyl Salicylat | | | | 6,00 |
| Homosalat | | | 5,00 | |
| Titandioxid Aluminium/Stearinsäure coating | 1,50 | | | |

(fortgesetzt)

| | 1 | 2 | 3 | 4 |
|---|---|---|---|---|
| Zinkoxid HP-1 | 2,00 | | | |
| Cocoyl amidopropyl Betain | | 4.00 | 2.00 | |
| Dimethicone Copolyol | | | 1.50 | |
| Decyl Glycoside | | 2.50 | | 2.00 |
| Natrium Laurylethersulfat SLES | | | 4,00 | 3,00 |
| Glycerin | 5,00 | | 2.00 | 3.20 |
| PVP/Hexadecen Copolymer | | 1.00 | | |
| Farbstoff (wasser- und/oder öllöslich) | | 0.20 | | 0.10 |
| Cyclomethicon | 3,00 | | | |
| Parabene | 1,50 | | | |
| Phenoxyethanol | | 0.20 | 0.15 | 0.10 |
| DMDM Hydantoin | | | 0.40 | |
| Natronlauge | q.s. | | | |
| Parfüm | q.s. | q.s. | q.s. | q.s. |
| Aqua | Ad 100 | Ad 100 | Ad 100 | Ad 100 |

## Patentansprüche

**1.** Kosmetische und dermatologische Emulsionen, enthaltend

A. ein oder mehrere Tenside,
B. eine oder mehrere pigmentäre organische Substanzen und
C. Pentanatrium-Ethylendiamintetramethylenphosphonat.

**2.** Verwendung von Pentanatrium-Ethylendiamintetramethylenphosphonat zum Erzielen oder Erhöhen der Stabilität von Emulsionen mit einem Gehalt an pigmentären organischen Substanzen gegenüber der Gegenwart von Tensiden.

**3.** Emulsionen nach Anspruch 1 oder Verwendung nach Anspruch 2, **dadurch gekennzeichnet, dass** die Emulsion eine O/W-Formulierung ist.

**4.** Emulsionen nach Anspruch 1 oder Verwendung nach Anspruch 2, **dadurch gekennzeichnet, dass** die Zubereitung mindestens eine UV-Filtersubstanz, gewählt aus der Gruppe Triazine, Campherderivate und organische und/oder anorganische Pigmente, enthält.

**5.** Emulsionen nach Anspruch 1 oder Verwendung nach Anspruch 2, **dadurch gekennzeichnet, dass** die Zubereitung mindestens eine UV-A-Filtersubstanz und/oder einen Breitbandfilter, gewählt aus der Gruppe Dibenzoylmethanderivate [insbesondere das 4-(tert.-Butyl)-4'-methoxydibenzoylmethan], 2,2'-Methylen-bis-(6-(2H-benzotriazol-2-yl)-4-(1,1,3,3-tetramethylbutyl)-phenol), 1,4-di(2-oxo-10-Sulfo-3-bornylidenmethyl)-Benzol und dessen Salze und 2,4-Bis-{[4-(2-Ethyl-hexyloxy)-2-hydroxy]-phenyl}-6-(4-methoxyphenyl)-1,3,5-triazin, enthält, wobei die weiteren Filtersubstanzen jeweils einzeln oder in beliebigen Kombinationen miteinander vorliegen können.

**6.** Emulsionen nach Anspruch 1 oder Verwendung nach Anspruch 2, **dadurch gekennzeichnet, dass** die Zubereitung mindestens ein Flavonglycosid, insbesondere α-Glucosylrutin, und/oder Vitamin E und/oder dessen Derivate enthält.

**7.** Verwendung von Zubereitungen nach Anspruch 5 oder 6 zur Herstellung eines Kosmetikums zum Schutz der Haut vor vorzeitiger Alterung.

**8.** Verwendung von Zubereitungen nach einem der vorhergehenden Ansprüche zur Herstellung eines Kosmetikums zur Hautbefeuchtung.

## Claims

**1.** Cosmetic and dermatological emulsions comprising

A. one or more surfactants,
B. one or more pigmentary organic substances and
C. pentasodium ethylenediaminetetramethylenephosphonate.

**2.** Use of pentasodium ethylenediaminetetramethylenephosphonate for achieving or increasing the stability of emulsions with a content of pigmentary organic substances towards the presence of surfactants.

**3.** Emulsions according to Claim 1 or use according to Claim 2, **characterized in that** the emulsion is an O/W formulation.

**4.** Emulsions according to Claim 1 or use according to Claim 2, **characterized in that** the preparation comprises at least one UV filter substance selected from the group of triazines, camphor derivatives and organic and/or inorganic pigments.

**5.** Emulsions according to Claim 1 or use according to Claim 2, **characterized in that** the preparation comprises at least one UV-A filter substance and/or a broadband filter selected from the group of dibenzoylmethane derivatives [in particular 4-(tert-butyl)-4'-methoxydibenzoylmethane], 2,2'-methylenebis(6-(2H-benzotriazol-2-yl)-4-(1,1,3,3-tetramethylbutyl)phenol), 1,4-di(2-oxo-10-sulpho-3-bornylidenemethyl)benzene and salts thereof and 2,4-bis{[4-(2-ethylhexyloxy)-2-hydroxy]phenyl)-6-(9-methoxyphenyl)-1,3,5-triazine, where the further filter substances may in each case be present individually or in any combinations with one another.

**6.** Emulsions according to Claim 1 or use according to Claim 2, **characterized in that** the preparation comprises at least one flavone glycoside, in particular $\alpha$-glucosylrutin, and/or vitamin E and/or derivatives thereof.

**7.** Use of preparations according to Claim 5 or 6 for producing a cosmetic for protecting the skin against premature ageing.

**8.** Use of preparations according to one of the preceding claims for producing a cosmetic for moisturizing the skin.

## Revendications

**1.** Emulsions cosmétiques et dermatologiques, contenant

A. un ou plusieurs tensioactifs,
B. une ou plusieurs substances organiques pigmentaires, et
C. de l'éthylènediaminetétraméthylènephosphonate pentasodique.

**2.** Utilisation de l'éthylènediaminetétraméthylènephosphonate pentasodique pour réaliser ou augmenter la stabilité d'émulsions contenant des substances organiques pigmentaires, par rapport à cette stabilité en présence de tensioactifs.

**3.** Emulsions selon la revendication 1, ou utilisation selon la revendication 2, **caractérisées en ce que** l'émulsion est une émulsion de type aqueux.

**4.** Emulsions selon la revendication 1 ou utilisation selon la revendication 2, **caractérisées en ce que** la préparation contient au moins une substance filtrant les UV, choisie dans le groupe des triazines, des dérivés du camphre et des pigments organiques et/ou inorganiques.

**5.** Emulsions selon la revendication 1 ou utilisation selon la revendication 2, **caractérisées en ce que** la préparation

contient au moins une substance filtrant les UV-A et/ou un filtre à large bande, choisis dans le groupe comprenant les dérivés du dibenzoylméthane [en particulier le 4-(tert-butyl)-4'-méthoxydibenzoylméthanel, le 2,2'-méthylène-bis(6-(2H-benzotriazol-2-yl)-4-(1,1,3,3-tétraméthylbutyl)-phénol), le 1,9-di(2-oxo-10-sulfo-3-bornylidèneméthyl) benzène et ses sels, et la 2,4-bis-([4-(2-éthyl-hexyloxy)-2-hydroxy]-phényl}-6-(4-méthoxyphényl)-1,3,5-triazine, les substances filtrantes supplémentaires pouvant chacune être présentes à titre individuel, ou selon des combinaisons quelconques les unes avec les autres.

**6.** Emulsions selon la revendication 1 ou utilisation selon la revendication 2, **caractérisées en ce que** la préparation contient au moins un flavone-glycoside, en particulier de l'α-glucosylrutzne, et/ou de la vitamine E et/ou ses dérivés.

**7.** Utilisation de préparations selon la revendication 5 ou 6 pour préparer un produit cosmétique destiné à protéger la peau d'un vieillissement prématuré.

**8.** Utilisation de préparations selon l'une des revendications précédentes, pour préparer un produit cosmétique destiné à l'hydratation de la peau.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 5352438 A
- US 6174520 B
- EP 0754446 A
- DE 4040655 A
- US 5663213 A
- EP 0761201 A


### In der Beschreibung aufgeführte Nicht-Patentliteratur


- *CHEMICAL ABSTRACTS,* 178463-23-5
- Rowe Colour Index. *Society of Dyers and Colourists,* 1971